# EUROPEAN PATENT APPLICATION

(11) **EP 3 269 730 A1**
(43) Date of publication of application: **17.01.2018**
(21) Application number: 16179032.4
(22) Date of filing: 12.07.2016
(51) Int. Cl.: C07K 14/435, A61K 39/35, C12N 15/00

(54) **ALLERGEN VARIANT**

(71) Applicant: Universität Salzburg, 5020 Salzburg (AT)
(72) Inventor: Wallner, Michael, 5071 Wals (AT); Ferreira, Fátima, 5020 Salzburg (AT); Lackner, Peter, 5020 Salzburg (AT); Hofer, Heidi, 5071 Wals (AT); Gadermaier, Gabriele, 5300 Hallwang (AT); Hauser, Michael, 6511 Zams (AT); Laimer, Josef, 5350 Strobl (AT)
(74) Representative: KLIMENT & HENHAPEL

(57) **Abstract**

The present invention relates to variants of a polypeptide of the Niemann-Pick type C2 (NPC2) protein family comprising at least one mutation.

SEQUENCE LISTING

<110>  Universität Salzburg

<120>  Allergen variant

<130>  47606

<160>  37

<170>  PatentIn version 3.5

<210>  1
<211>  31
<212>  PRT
<213>  Artificial Sequence

<220>
<223>  consensus sequence


<220>
<221>  MISC_FEATURE
<222>  (3)..(3)
<223>  Xaa = X1 is N or D

<220>
<221>  MISC_FEATURE
<222>  (4)..(4)
<223>  Xaa = X2 is S, SE or T

<220>
<221>  MISC_FEATURE
<222>  (5)..(5)
<223>  Xaa = X3 is K, N, A, P or E

<220>
<221>  MISC_FEATURE
<222>  (6)..(6)
<223>  Xaa = X4 is a peptide consisting of 15, 16 or 17 amino acid
       residues

<220>
<221>  MISC_FEATURE
<222>  (7)..(7)
<223>  Xaa = X5 is V or I

<220>
<221>  MISC_FEATURE
<222>  (10)..(10)
<223>  Xaa = X6 is a peptide consisting of 3 amino acid residues

<220>
<221>  MISC_FEATURE
<222>  (11)..(11)
<223>  Xaa =  X7 is N or D

<220>
<221>  MISC_FEATURE
<222>  (12)..(12)
<223>  Xaa =  X8 is A, L or G

<220>

<221>  MISC_FEATURE
<222>  (14)..(14)
<223>  Xaa =  X9  is K or H

<220>
<221>  MISC_FEATURE
<222>  (15)..(15)
<223>  Xaa =  X10 is a peptide consisting of 3 amino acid residues

<220>
<221>  MISC_FEATURE
<222>  (18)..(18)
<223>  Xaa =  X11 is a peptide consisting of 2 amino acid residues

<220>
<221>  MISC_FEATURE
<222>  (19)..(19)
<223>  Xaa =  X12 is K

<220>
<221>  MISC_FEATURE
<222>  (21)..(21)
<223>  Xaa =  X13 is a peptide consisting of 6 amino acid residues

<220>
<221>  MISC_FEATURE
<222>  (23)..(23)
<223>  Xaa =  X14 is T, S, A, D, N or NI

<220>
<221>  MISC_FEATURE
<222>  (24)..(24)
<223>  Xaa =  X15 is a peptide consisting of 2 amino acid residues or G,
       L, Y or W

<220>
<221>  MISC_FEATURE
<222>  (25)..(25)
<223>  Xaa =  X16 is N, V, T, L, I, S or E

<220>
<221>  MISC_FEATURE
<222>  (27)..(27)
<223>  Xaa =  X17 is K, R, A, Q or T

<220>
<221>  MISC_FEATURE
<222>  (28)..(28)
<223>  Xaa =  X18 is I, L, V, N or A

<220>
<221>  MISC_FEATURE
<222>  (29)..(29)
<223>  Xaa =  X19 is I, L, T or A

<220>
<221>  MISC_FEATURE
<222>  (31)..(31)
<223>  Xaa =  X20 is K, N, S, T or E

<400>  1

Asn Gln Xaa Xaa Xaa Xaa Xaa Pro Gly Xaa Xaa Xaa Cys Xaa Xaa Cys

1               5                   10                  15

Pro Xaa Xaa Gly Xaa Tyr Xaa Xaa Xaa Pro Xaa Xaa Xaa Pro Xaa
            20                  25                  30

<210>  2
<211>  57
<212>  PRT
<213>  Artificial Sequence

<220>
<223>  motif

<400>  2

Asn Gln Asn Ser Lys Thr Ala Lys Ile Glu Ile Lys Ala Ser Ile Asp
1               5                   10                  15

Gly Leu Glu Val Asp Val Pro Gly Ile Asp Pro Asn Ala Cys His Tyr
            20                  25                  30

Met Lys Cys Pro Leu Val Lys Gly Gln Gln Tyr Asp Ile Lys Tyr Thr
        35                  40                  45

Trp Asn Val Pro Lys Ile Ala Pro Lys
    50                  55

<210>  3
<211>  59
<212>  PRT
<213>  Artificial Sequence

<220>
<223>  motif

<400>  3

Asn Gln Asp Thr Ala Ser Val Gly Leu Val Leu Thr Ala Ile Ile Asp
1               5                   10                  15

Gly Asp Ile Ala Ile Asp Ile Pro Gly Leu Glu Thr Asn Ala Cys Lys
            20                  25                  30

Leu Met Lys Cys Pro Ile Arg Lys Gly Glu His Gln Glu Leu Ile Tyr
        35                  40                  45

Asn Ile Gly Glu Ile Pro Asp Ala Thr Pro Glu
    50                  55

<210>  4
<211>  57
<212>  PRT
<213>  Artificial Sequence

<220>
<223>  motif

<400>  4

Asn Gln Asp Thr Ala Lys Val Thr Ile Lys Val Leu Ala Lys Val Ala
1               5                   10                  15

Gly Thr Thr Ile Gln Val Pro Gly Leu Glu Thr Asp Gly Cys Lys Phe
            20                  25                  30

Ile Lys Cys Pro Val Lys Lys Gly Glu Ala Leu Asp Phe Ile Tyr Ser
        35                  40                  45

Gly Thr Ile Pro Ala Ile Thr Pro Lys
    50                  55

<210>  5
<211>  57
<212>  PRT
<213>  Artificial Sequence

<220>
<223>  motif

<400>  5

Asn Gln Asp Ser Pro Thr Ala Val Ile Lys Ile Ser Ala Lys Val Asn
1               5                   10                  15

Gly Val Glu Leu Gln Val Pro Gly Ile Glu Thr Asn Gly Cys His His
            20                  25                  30

Met Lys Cys Pro Leu Val Lys Gly Gln Ser Tyr Gln Phe Lys Tyr Asp
        35                  40                  45

Leu Val Ile Pro Gln Ile Leu Pro Asn
    50                  55

<210>  6
<211>  57
<212>  PRT
<213>  Artificial Sequence

<220>
<223>  motif

<400>  6

Asn Gln Asp Ser Glu Lys Leu Glu Ile Lys Ile Ser Ala Ile Met Asn
1               5                   10                  15

Asn Ile Glu Val Pro Val Pro Gly Val Asp Lys Asp Gly Cys Lys His

20                  25                  30

Thr Thr Cys Pro Leu Lys Lys Gly Gln Lys Tyr Glu Leu Asp Tyr Ser
        35                  40                  45

Leu Ile Ile Pro Thr Ile Leu Pro Asn
    50                  55

<210>  7
<211>  57
<212>  PRT
<213>  Artificial Sequence

<220>
<223>  motif

<400>  7

Asn Gln Asp Ser Ala Lys Ile Glu Val His Leu Thr Ala Asn Ile Asp
1               5                   10                  15

Gly Leu Ser Ile Pro Ile Pro Gly Val Asp Lys Asp Gly Cys Lys Tyr
            20                  25                  30

Val Thr Cys Pro Ile Lys Lys Gly Glu Lys Val His Phe Asn Tyr Ser
        35                  40                  45

Leu Val Val Pro Lys Leu Ile Pro Asn
    50                  55

<210>  8
<211>  57
<212>  PRT
<213>  Artificial Sequence

<220>
<223>  motif

<400>  8

Asn Gln Asp Thr Ala Lys Ile Ser Val His Leu Ser Ala Lys Val Glu
1               5                   10                  15

Gly Leu Glu Ile Pro Ile Pro Gly Val Asp Arg Asp Gly Cys Lys Tyr
            20                  25                  30

Val Lys Cys Pro Val Lys Lys Gly Glu His Leu His Leu Asn Tyr Ala
        35                  40                  45

Leu Thr Val Pro Lys Leu Leu Pro Asn
    50                  55

<210>  9
<211>  57
<212>  PRT
<213>  Artificial Sequence

<220>
<223>  motif

<400>  9

Asn Gln Asp Thr Ala His Val Lys Ile Gln Leu Thr Ala Ser Met Gly
1               5                   10                  15

Gly Ile Glu Ile Pro Val Pro Gly Val Gly Thr Asp Gly Cys Lys Tyr
            20                  25                  30

Ile Lys Cys Pro Leu Val Lys Gly Glu Lys Lys Thr Phe Pro Tyr Thr
        35                  40                  45

Tyr Leu Val Pro Lys Ile Leu Pro Ser
    50                  55

<210>  10
<211>  57
<212>  PRT
<213>  Artificial Sequence

<220>
<223>  motif

<400>  10

Asn Gln Asp Ser Ala Asn Leu Lys Leu Asp Ile Val Ala Asp Ile Asn
1               5                   10                  15

Gly Val Gln Ile Glu Val Pro Gly Val Asp His Asp Gly Cys His Tyr
            20                  25                  30

Val Lys Cys Pro Ile Lys Lys Gly Gln His Phe Asp Val Lys Tyr Thr
        35                  40                  45

Tyr Ser Ile Pro Ala Ile Leu Pro Thr
    50                  55

<210>  11
<211>  57
<212>  PRT
<213>  Artificial Sequence

<220>
<223>  motif

<400>  11

Asn Gln Asn Thr Lys Thr Ala Lys Ile Glu Ile Lys Ala Ser Leu Asp

1               5                   10                  15

Gly Leu Glu Ile Asp Val Pro Gly Ile Asp Thr Asn Ala Cys His Phe
            20                  25                  30

Met Lys Cys Pro Leu Val Lys Gly Gln Gln Tyr Asp Ile Lys Tyr Thr
        35                  40                  45

Trp Asn Val Pro Lys Ile Ala Pro Lys
    50                  55

<210>  12
<211>  57
<212>  PRT
<213>  Artificial Sequence

<220>
<223>  motif

<400>  12

Asn Gln Asn Ser Asn Ala Ala Lys Ile Glu Ile Lys Ala Thr Ile Asp
1               5                   10                  15

Gly Val Glu Ile Asp Val Pro Gly Ile Asp Asn Asn Leu Cys His Phe
            20                  25                  30

Met Lys Cys Pro Leu Val Lys Gly Gln Glu Tyr Asp Ile Lys Tyr Thr
        35                  40                  45

Trp Asn Val Pro Arg Ile Ala Pro Lys
    50                  55

<210>  13
<211>  9
<212>  PRT
<213>  Artificial Sequence

<220>
<223>  consensus sequence

<220>
<221>  MISC_FEATURE
<222>  (1)..(1)
<223>  Xaa = X21 is L or I

<220>
<221>  MISC_FEATURE
<222>  (3)..(3)
<223>  Xaa = X22 is R or K

<220>
<221>  MISC_FEATURE
<222>  (5)..(5)

<223>  Xaa = X23 is K, T or E

<220>
<221>  MISC_FEATURE
<222>  (6)..(6)
<223>  Xaa = X24 is K, P, A, E or S

<220>
<221>  MISC_FEATURE
<222>  (7)..(7)
<223>  Xaa = X25 is F, L or M

<220>
<221>  MISC_FEATURE
<222>  (8)..(8)
<223>  Xaa = X26 is Q, K, A, T, S or D

<220>
<221>  MISC_FEATURE
<222>  (9)..(9)
<223>  Xaa = X27 is L or M

<400>  13

Xaa His Xaa Gly Xaa Xaa Xaa Xaa Xaa
1               5


<210>  14
<211>  8
<212>  PRT
<213>  Artificial Sequence

<220>
<223>  motif

<400>  14

His Arg Gly Lys Pro Phe Gln Leu
1               5


<210>  15
<211>  8
<212>  PRT
<213>  Artificial Sequence

<220>
<223>  motif

<400>  15

His Arg Gly Lys Pro Leu Thr Leu
1               5


<210>  16
<211>  8
<212>  PRT
<213>  Artificial Sequence

<220>
<223>  motif

<400>  16

His Arg Gly Glu Lys Met Thr Leu
1               5


<210>  17
<211>  8
<212>  PRT
<213>  Artificial Sequence

<220>
<223>  motif

<400>  17

His Lys Gly Lys Lys Leu Ser Met
1               5


<210>  18
<211>  8
<212>  PRT
<213>  Artificial Sequence

<220>
<223>  motif

<400>  18

His Lys Gly Lys Ser Phe Thr Leu
1               5


<210>  19
<211>  8
<212>  PRT
<213>  Artificial Sequence

<220>
<223>  motif

<400>  19

His Lys Gly Lys Glu Leu Lys Leu
1               5


<210>  20
<211>  8
<212>  PRT
<213>  Artificial Sequence

<220>
<223>  motif

<400>  20

His Lys Gly Lys Pro Leu Ala Leu
1               5

<210>  21
<211>  8
<212>  PRT
<213>  Artificial Sequence

<220>
<223>  motif

<400>  21

His Lys Gly Lys Glu Leu Lys Leu
1               5


<210>  22
<211>  8
<212>  PRT
<213>  Artificial Sequence

<220>
<223>  motif

<400>  22

His Lys Gly Lys Lys Leu Asp Leu
1               5


<210>  23
<211>  8
<212>  PRT
<213>  Artificial Sequence

<220>
<223>  motif

<400>  23

His Arg Gly Lys Pro Phe Thr Leu
1               5


<210>  24
<211>  8
<212>  PRT
<213>  Artificial Sequence

<220>
<223>  motif

<400>  24

His Arg Gly Thr Ala Phe Gln Leu
1               5


<210>  25
<211>  129
<212>  PRT
<213>  Artificial Sequence

<220>
<223>  allergen fragment

<400>  25

Asp Gln Val Asp Val Lys Asp Cys Ala Asn His Glu Ile Lys Lys Val
1               5                   10                  15

Leu Val Pro Gly Cys His Gly Ser Glu Pro Cys Ile Ile His Arg Gly
            20                  25                  30

Lys Pro Phe Gln Leu Glu Ala Leu Phe Glu Ala Asn Gln Asn Ser Lys
        35                  40                  45

Thr Ala Lys Ile Glu Ile Lys Ala Ser Ile Asp Gly Leu Glu Val Asp
    50                  55                  60

Val Pro Gly Ile Asp Pro Asn Ala Cys His Tyr Met Lys Cys Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Val Met Gly
            100                 105                 110

Asp Asn Gly Val Leu Ala Cys Ala Ile Ala Thr His Ala Lys Ile Arg
        115                 120                 125

Asp

<210>  26
<211>  128
<212>  PRT
<213>  Artificial Sequence

<220>
<223>  allergen fragment

<400>  26

Gly Lys Met Asn Phe Thr Asp Cys Gly His Asn Glu Ile Lys Glu Leu
1               5                   10                  15

Ser Val Ser Asn Cys Thr Gly Asn Tyr Cys Val Ile His Arg Gly Lys
            20                  25                  30

Pro Leu Thr Leu Asp Ala Lys Phe Asp Ala Asn Gln Asp Thr Ala Ser
        35                  40                  45

Val Gly Leu Val Leu Thr Ala Ile Ile Asp Gly Asp Ile Ala Ile Asp
    50                  55                  60

Ile Pro Gly Leu Glu Thr Asn Ala Cys Lys Leu Met Lys Cys Pro Ile
65                  70                  75                  80

Arg Lys Gly Glu His Gln Glu Leu Ile Tyr Asn Ile Gly Glu Ile Pro
                85                  90                  95

Asp Ala Thr Pro Glu Ile Lys Ala Lys Val Lys Ala Gln Leu Ile Gly
            100                 105                 110

Glu His Gly Val Leu Ala Cys Gly Trp Val Asp Gly Glu Val Gln Glu
        115                 120                 125

<210>  27
<211>  125
<212>  PRT
<213>  Artificial Sequence

<220>
<223>  allergen fragment

<400>  27

Gly Lys Met Thr Phe Lys Asp Cys Gly His Gly Glu Val Thr Glu Leu
1               5                   10                  15

Asp Ile Thr Gly Cys Ser Gly Asp Thr Cys Val Ile His Arg Gly Glu
            20                  25                  30

Lys Met Thr Leu Glu Ala Lys Phe Ala Ala Asn Gln Asp Thr Ala Lys
        35                  40                  45

Val Thr Ile Lys Val Leu Ala Lys Val Ala Gly Thr Thr Ile Gln Val
    50                  55                  60

Pro Gly Leu Glu Thr Asp Gly Cys Lys Phe Ile Lys Cys Pro Val Lys
65                  70                  75                  80

Lys Gly Glu Ala Leu Asp Phe Ile Tyr Ser Gly Thr Ile Pro Ala Ile
                85                  90                  95

Thr Pro Lys Val Lys Ala Asp Val Thr Ala Glu Leu Ile Gly Asp His
            100                 105                 110

Gly Val Met Ala Cys Gly Thr Val His Gly Gln Val Glu
        115                 120                 125

<210>  28
<211>  126
<212>  PRT

<213>  Artificial Sequence

<220>
<223>  allergen fragment

<400>  28

Gly Glu Met Lys Phe Gln Asp Cys Gly His Gly Glu Val Lys Lys Leu
1               5                   10                  15

Leu Val Ser Asp Cys Ser Gly Asp Tyr Cys Ile Ile His Lys Gly Lys
            20                  25                  30

Lys Leu Ser Met Glu Ala Asp Phe Val Ala Asn Gln Asp Ser Pro Thr
        35                  40                  45

Ala Val Ile Lys Ile Ser Ala Lys Val Asn Gly Val Glu Leu Gln Val
    50                  55                  60

Pro Gly Ile Glu Thr Asn Gly Cys His His Met Lys Cys Pro Leu Val
65                  70                  75                  80

Lys Gly Gln Ser Tyr Gln Phe Lys Tyr Asp Leu Val Ile Pro Gln Ile
                85                  90                  95

Leu Pro Asn Val Lys Ala Asp Val Thr Ala Ser Leu Thr Gly Ala His
            100                 105                 110

Gly Leu Leu Ala Cys Gly Thr Val His Gly Glu Val Gln Asn
        115                 120                 125


<210>  29
<211>  125
<212>  PRT
<213>  Artificial Sequence

<220>
<223>  allergen fragment

<400>  29

Arg Cys Gln Ile Tyr Arg Ser Cys Thr Trp Glu Val Thr Ser Leu Asp
1               5                   10                  15

Leu Ser Gly Cys Ser Gly Asp His Cys Ile Ile His Lys Gly Lys Ser
            20                  25                  30

Phe Thr Leu Lys Thr Phe Phe Ile Ala Asn Gln Asp Ser Glu Lys Leu
        35                  40                  45

Glu Ile Lys Ile Ser Ala Ile Met Asn Asn Ile Glu Val Pro Val Pro
    50                  55                  60

Gly Val Asp Lys Asp Gly Cys Lys His Thr Thr Cys Pro Leu Lys Lys
65                  70                  75                  80

Gly Gln Lys Tyr Glu Leu Asp Tyr Ser Leu Ile Ile Pro Thr Ile Leu
                85                  90                  95

Pro Asn Leu Lys Thr Val Thr Thr Ala Ser Leu Val Gly Asp His Gly
            100                 105                 110

Val Val Ala Cys Gly Lys Val Asn Thr Glu Val Val Asp
        115                 120                 125

<210>  30
<211>  129
<212>  PRT
<213>  Artificial Sequence

<220>
<223>  allergen fragment

<400>  30

Gly Gln Val Arg Phe Glu Asp Cys Gly His His Glu Val Thr Lys Leu
1               5                   10                  15

Asp Ile Ser Gln Cys Ala Asp Gly Ala Ser Thr Cys Val Ile His Lys
            20                  25                  30

Gly Lys Glu Leu Lys Leu Asp Ala Glu Ile Leu Ala Asn Gln Asp Ser
        35                  40                  45

Ala Lys Ile Glu Val His Leu Thr Ala Asn Ile Asp Gly Leu Ser Ile
    50                  55                  60

Pro Ile Pro Gly Val Asp Lys Asp Gly Cys Lys Tyr Val Thr Cys Pro
65                  70                  75                  80

Ile Lys Lys Gly Glu Lys Val His Phe Asn Tyr Ser Leu Val Val Pro
                85                  90                  95

Lys Leu Ile Pro Asn Leu His Asn Val Glu Val Gly Ala Lys Ile Thr
            100                 105                 110

Gly Asp His Gly Leu Leu Ala Cys Leu Arg Leu Lys Gly Asp Ile Ala
        115                 120                 125

Asn

<210>  31
<211>  129
<212>  PRT
<213>  Artificial Sequence

<220>
<223>  allergen fragment

<400>  31

Gly Asn Val Lys Phe Glu Asp Cys Gly His His Glu Val Val Lys Leu
1               5                   10                  15

Asn Ile Ser Gln Cys Ala Asp Gly Val Ala Thr Cys Val Leu His Lys
            20                  25                  30

Gly Lys Pro Leu Ala Leu Asp Ala Glu Met Ile Ser Asn Gln Asp Thr
        35                  40                  45

Ala Lys Ile Ser Val His Leu Ser Ala Lys Val Glu Gly Leu Glu Ile
    50                  55                  60

Pro Ile Pro Gly Val Asp Arg Asp Gly Cys Lys Tyr Val Lys Cys Pro
65                  70                  75                  80

Val Lys Lys Gly Glu His Leu His Leu Asn Tyr Ala Leu Thr Val Pro
                85                  90                  95

Lys Leu Leu Pro Asn Leu His Asn Val Glu Ile Gly Ala Lys Ile Ser
            100                 105                 110

Gly Asp His Gly Leu Leu Ala Cys Leu Arg Leu His Gly Asp Leu Ser
        115                 120                 125

Asn

<210>  32
<211>  127
<212>  PRT
<213>  Artificial Sequence

<220>
<223>  allergen fragment

<400>  32

Thr Pro Ile Pro Tyr Thr Asp Cys Gly Asn His Glu Leu Ser Lys Leu
1               5                   10                  15

Asp Ala Asn Leu His Cys Ala Gly Asp Leu Cys Thr Ile His Lys Gly
            20                  25                  30

Lys Glu Leu Lys Leu Asp Ala Gln Phe Thr Val Asn Gln Asp Thr Ala
        35                  40                  45

His Val Lys Ile Gln Leu Thr Ala Ser Met Gly Gly Ile Glu Ile Pro
    50                  55                  60

Val Pro Gly Val Gly Thr Asp Gly Cys Lys Tyr Ile Lys Cys Pro Leu
65                  70                  75                  80

Val Lys Gly Glu Lys Lys Thr Phe Pro Tyr Thr Tyr Leu Val Pro Lys
                85                  90                  95

Ile Leu Pro Ser Val Thr Gly Asp Val Lys Ala Val Phe Thr Gly Asp
            100                 105                 110

His Gly Pro Leu Gly Cys Leu Lys Phe His Gly Lys Ile Val Asp
        115                 120                 125

<210>  33
<211>  126
<212>  PRT
<213>  Artificial Sequence

<220>
<223>  allergen fragment

<400>  33

Gly Lys Val Lys Phe Gln Asp Cys Gly Lys Gly Glu Val Glu Ser Leu
1               5                   10                  15

Glu Val Glu Gly Cys Ser Gly Asp Tyr Cys Val Ile His Lys Gly Lys
            20                  25                  30

Lys Leu Asp Leu Ala Ile Ser Val Thr Ser Asn Gln Asp Ser Ala Asn
        35                  40                  45

Leu Lys Leu Asp Ile Val Ala Asp Ile Asn Gly Val Gln Ile Glu Val
    50                  55                  60

Pro Gly Val Asp His Asp Gly Cys His Tyr Val Lys Cys Pro Ile Lys
65                  70                  75                  80

Lys Gly Gln His Phe Asp Val Lys Tyr Thr Tyr Ser Ile Pro Ala Ile
                85                  90                  95

Leu Pro Thr Thr Lys Ala Lys Ile Ile Ala Lys Ile Ile Gly Asp Lys
            100                 105                 110

Gly Leu Gly Gly Cys Ile Val Ile Asn Gly Glu Ile Gln Asp
        115                 120                 125

<210>  34
<211>  129
<212>  PRT
<213>  Artificial Sequence

<220>
<223>  allergen fragment

<400>  34

Asp Gln Val Asp Val Lys Asp Cys Ala Asn Asn Glu Ile Lys Lys Val
1               5                   10                  15

Met Val Asp Gly Cys His Gly Ser Asp Pro Cys Ile Ile His Arg Gly
            20                  25                  30

Lys Pro Phe Thr Leu Glu Ala Leu Phe Asp Ala Asn Gln Asn Thr Lys
        35                  40                  45

Thr Ala Lys Ile Glu Ile Lys Ala Ser Leu Asp Gly Leu Glu Ile Asp
    50                  55                  60

Val Pro Gly Ile Asp Thr Asn Ala Cys His Phe Met Lys Cys Pro Leu
65                  70                  75                  80

Val Lys Gly Gln Gln Tyr Asp Ile Lys Tyr Thr Trp Asn Val Pro Lys
                85                  90                  95

Ile Ala Pro Lys Ser Glu Asn Val Val Val Thr Val Lys Leu Ile Gly
            100                 105                 110

Asp Asn Gly Val Leu Ala Cys Ala Ile Ala Thr His Gly Lys Ile Arg
        115                 120                 125

Asp

<210>  35
<211>  129
<212>  PRT
<213>  Artificial Sequence

<220>
<223>  allergen fragment

<400>  35

Asp Gln Val Asp Ile Lys Asp Cys Ala Asn His Glu Ile Lys Lys Val
1               5                   10                  15

Met Val Pro Gly Cys Lys Gly Ser Glu Pro Cys Val Ile His Arg Gly
            20                  25                  30

Thr Ala Phe Gln Leu Glu Ala Val Phe Asp Ala Asn Gln Asn Ser Asn
        35                  40                  45

Ala Ala Lys Ile Glu Ile Lys Ala Thr Ile Asp Gly Val Glu Ile Asp
    50                  55                  60

Val Pro Gly Ile Asp Asn Asn Leu Cys His Phe Met Lys Cys Pro Leu
65                  70                  75                  80

Val Lys Gly Gln Glu Tyr Asp Ile Lys Tyr Thr Trp Asn Val Pro Arg
                85                  90                  95

Ile Ala Pro Lys Ser Glu Asn Val Val Val Thr Val Lys Leu Leu Gly
            100                 105                 110

Asp Asn Gly Val Leu Ala Cys Ala Ile Ala Thr His Ala Lys Ile Arg
        115                 120                 125

Asp

<210> 36
<211> 129
<212> PRT
<213> Artificial Sequence

<220>
<223> allergen variant

<400> 36

Asp Gln Val Asp Val Lys Asp Cys Ala Asn His Glu Ile Lys Lys Val
1               5                   10                  15

Leu Val Pro Gly Cys His Gly Ser Glu Pro Cys Ile Ile His Arg Gly
            20                  25                  30

Lys Pro Phe Lys Leu Glu Ala Leu Phe Glu Ala Asn Gln Asn Ser Thr
        35                  40                  45

Thr Ala Lys Ile Glu Ile Lys Ala Ser Ile Asp Gly Leu Glu Val Asp
    50                  55                  60

Val Pro Gly Ile Asp Pro Asn Ala Cys Glu Tyr Met Lys Cys Pro Leu
65                  70                  75                  80

Val Asp Gly Gln Gln Tyr Asp Ile Lys Tyr Glu Trp Asp Val Pro Glu
                85                  90                  95

Ile Ala Pro Gln Ser Glu Asn Val Val Val Thr Val Lys Val Met Gly
            100                 105                 110

Asp Asn Gly Val Leu Ala Cys Ala Ile Ala Thr His Ala Lys Ile Arg
        115                 120                 125

Asp

<210> 37
<211> 126
<212> PRT
<213> Artificial Sequence

<220>
<223> allergen variant

<400> 37

Gly Asp Val Lys Phe Thr Asp Cys Ala His Gly Glu Val Thr Ser Leu
1               5                   10                  15

Asp Leu Ser Gly Cys Ser Gly Asp His Cys Thr Ile His Lys Gly Lys
            20                  25                  30

Ser Phe Thr Leu Lys Thr Phe Phe Ile Ala Asn Gln Asp Ser Glu Lys
        35                  40                  45

Leu Glu Ile Lys Ile Ser Ala Thr Met Asn Gly Ile Glu Val Pro Val
    50                  55                  60

Pro Gly Val Asp Lys Asp Gly Cys Lys His Thr Thr Cys Pro Leu Lys
65                  70                  75                  80

Lys Gly Gln Lys Tyr Glu Leu Asp Tyr Ser Leu Ile Ile Pro Thr Val
                85                  90                  95

Leu Pro Asn Val Lys Thr Val Thr Thr Ala Ser Leu Val Gly Asp His
            100                 105                 110

Gly Val Val Ala Cys Gly Lys Val Asn Thr Glu Val Val Asp
        115                 120                 125

## Description

The present invention relates to variants of polypeptides of the Niemann-Pick type C2 (NPC2) protein family.

Allergens of the Niemann-Pick type C2 (NPC2) protein family, in particular allergens of mites like house dust mites (HDM) and storage mites are present almost all over the world, extending from the temperate climate zones to the tropics. Mite feces contain various allergens, which can not only frequently raise allergic symptoms in patients, but they also bear a high risk for patients to develop allergic asthma. Worldwide, *Dermatophagoides pteronyssinus* is the most prevalent house dust mite species, followed by *Dermatophagoides farina* (Calderon et al., J Allergy Clin Immunol. 2015 Jul;136(1):38-48*.).* Both species are highly related and also cross-reactivity between their allergens is observed. The most common allergy eliciting proteins belong to the group 1 and 2 allergens, showing sensitization rates of 60% to 100% among mite allergic patients.

As the risk for developing asthma in HDM sensitized patients is high, allergy treatment by allergen immunotherapy (AIT) has been suggested as alternative to anti-histamine- or corticosteroid-based treatment strategies. Up to date, crude extracts are the active compound in AIT vaccine formulations for HDM therapy. Even though extracts can be produced fairly inexpensive and in relatively large quantities, they show several deficiencies such as problems in the standardization and characterization process, and possible batch-to-batch variations. Moreover, it has been reported that some extracts do not contain sufficient amounts of allergen. Long treatment schedules in AIT dependent on repeated injections for a period of up to 5 years leads to a high dropout rate resulting in poor treatment efficacy. Further, mild side effects, such as local swelling or itching, but also systemic reactions can occur during AIT. Therefore, several approaches have been proposed to improve AIT.

For instance, instead of using highly variable extracts, recombinantly produced allergens have been investigated. The recombinant production of allergens has the advantage that the allergen of interest can be produced in relatively large amounts and in a relatively pure form. However, recombinantly produced allergens as well as allergens isolated from natural sources usually maintain their allergenic properties so that the administration of such molecules and mixtures may cause unwanted side effects. In order to avoid these side effects it is advantageous to reduce significantly or entirely these allergenic properties keeping their capability to induce an allergen specific immune response. Since the IgE binding capacity of allergens is responsible for their allergenic properties the reduction of IgE binding sites on allergens is most important.

Up to now several strategies to reduce the allergenic properties (i.e. IgE binding capacity) of allergens have been described. Fragmentation of an allergen, for instance, is often used to destroy its IgE binding sites whereby its T cell epitopes, which are required for a T cell driven immune response, are maintained. Other methods to reduce the IgE binding capacity of an allergenic polypeptide involve the introduction of point mutations within the allergen. Point mutations have the advantage that the overall three-dimensional structure of the allergen can be kept although its IgE binding capacity is significantly reduced.

It is an object of the present invention to provide variants of allergens, which exhibit a reduced IgE binding capacity, and can therefore be used for preventing and treating individuals suffering from allergies.

Therefore, the present invention relates to a variant of a polypeptide of the Niemann-Pick type C2 (NPC2) protein family comprising a motif consisting of the amino acid sequence NQX₁X₂X₃X₄X₅PGX₆X₇X₈CX₉X₁₀CPX₁₁X₁₂GX₁₃YX₁₄X₁₅X₁₆PX₁₇X₁₈X₁₉PX₂₀ (SEQ ID No. 1), wherein X₁ is N or D, X₂ is S or T, X₃ is K, N, A, P or E, X₄ is a peptide consisting of 16 or 17 amino acid residues, X₅ is V or I, X₆ is a peptide consisting of 3 amino acid residues, X₇ is N or D, X₈ is A, L or G, X₉ is H or K, X₁₀ is a peptide consisting of 3 amino acid residues, X₁₁ is a peptide consisting of 2 amino acid residues, X₁₂ is K, X₁₃ is a peptide consisting of 6 amino acid residues, X₁₄ is either a single amino acid such as T, S, A, D or N or two amino acids such as NI, X₁₅ is a peptide consisting of 2 amino acid residues or G, L, Y or W, X₁₆ is N, V, T, L, I, S or E, X₁₇ is K, R, A, Q or T, X₁₈ is I, L, V, N or A, X₁₉ is I, L, T or A and X₂₀ is K, N, S, T or E, wherein at least one amino acid residue selected from the group consisting of X₃, X₉, X₁₂, X₁₇ and X₂₀ is mutated.

The Niemann-Pick type C2 (NPC2) protein family (also known as Epididymal secretory protein E1 family) comprises several members, which are known to be allergens. These allergens are derived from mites like *Acarus siro* (e.g. Aca s 2), *Aleuroglyphus ovatus* (e.g. Ale o 2), *Blomia tropicalis* (e.g. Blo t 2 and its isoforms), *Dermatophagoides farina* (e.g. Der f 2 and its isoforms), *Dermatophagoides pteronyssinus* (e.g. Der p 2 and its isoforms), *Euroglyphus maynei* (e.g. Eur m 2 and its isoforms), *Glycyphagus domesticus* (e.g. Gly d 2 and its isoforms), *Lepido-glyphus destructor* (e.g. Lep d 2 and its isoforms), *Psoroptes ovis* (e.g. Pso o 2), *Suidasia medanensis* (e.g. Sui m 2) and *Tyrophagus communis* (e.g. Tyr p 2 and its isoforms) and mammals like dogs *(Canis familiaris,* e.g. Can f 7 and its isoforms). The members of the NPC2 family are characterized by the presence of a motif consisting of the amino acid sequence NQX₁X₂X₃X₄X₅PGX₆X₇X₈CX₉X₁₀CPX₁₁X₁₂GX₁₃YX₁₄X₁₅X₁₆PX₁₇X₁₈X₁₉PX₂₀ (SEQ ID No. 1). It surprisingly turned out that the allergenicity of allergens of the NPC2 family can be significantly or entirely reduced if one or more amino acid residues of the respective allergens are mutated. According to the present invention at least one amino acid residue selected from the group consisting of X₃, X₉, X₁₂, X₁₇ and X₂₀ of the motif having the amino acid sequence SEQ ID No. 1 has to be mutated in order to obtain an allergen variant exhibiting a reduced allergenicity. Therefore, the variant of the present invention exhibits preferably a reduced allergenic activity (IgE binding capacity) compared to the unmodified and unmutated polypeptide of the Niemann-Pick type C2 (NPC2) protein family comprising a motif consisting of SEQ ID No. 1.

In a preferred embodiment of the present invention at least two, preferably at least three, more preferably at least four, in particular all five, amino acid residue selected from the group consisting of X₃, X₉, X₁₂, X₁₇ and X₂₀ of SEQ ID No. 1 are mutated.

According to a particular preferred embodiment of the present invention amino acid residues X₃, X₉, X₁₂, X₁₇ and X₂₀ of SEQ ID No. 1 are mutated.

Herein, the three- and one-letter code to denominate amino acid residues is used:

| **Amino Acid** | **3-Letter** | **1-Letter** | **Side-chain polarity** | **Side-chain charge (pH 7.4)** |
|---|---|---|---|---|
| Alanine | Ala | A | nonpolar | neutral |
| Arginine | Arg | R | basic polar | positive |
| Asparagine | Asn | N | polar | neutral |
| Aspartic acid | Asp | D | acidic polar | negative |
| Cysteine | Cys | C | nonpolar | neutral |
| Glutamic acid | Glu | E | acidic polar | negative |
| Glutamine | Gln | Q | polar | neutral |
| Glycine | Gly | G | nonpolar | neutral |
| Histidine | His | H | basic polar | positive(10%) neutral(90%) |
| Isoleucine | Ile | I | nonpolar | neutral |
| Leucine | Leu | L | nonpolar | neutral |
| Lysine | Lys | K | basic polar | positive |
| Methionine | Met | M | nonpolar | neutral |
| Phenylalanine | Phe | F | nonpolar | neutral |
| Proline | Pro | P | nonpolar | neutral |
| Serine | Ser | S | polar | neutral |
| Threonine | Thr | T | polar | neutral |
| Tryptophan | Trp | W | nonpolar | neutral |
| Tyrosine | Tyr | Y | polar | neutral |
| Valine | Val | V | nonpolar | neutral |

The term "variant", as used herein, refers to a polypeptide that differs from a naturally occurring (wild-type) polypeptide of the Niemann-Pick type C2 (NPC2) protein family by one or more amino acid residues. The variant includes modifications of the amino acid sequence by substituting, deleting or adding one or more amino acid residues, preferably naturally occurring amino acid residues, of the wild-type polypeptide. The two- and/or three-dimensional structure of the variant of the present invention is preferably at least substantially retained compared to the wild-type polypeptide.

"A polypeptide of the Niemann-Pick type C2 (NPC2) protein family", as defined herein, comprises a motif consisting of the amino acid sequence
NQX₁X₂X₃X₄X₅PGX₆X₇X₈CX₉X₁₀CPX₁₁X₁₂GX₁₃YX₁₄X₁₅X₁₆PX₁₇X₁₈X₁₉PX₂₀ (SEQ ID No. 1) and members of this protein family are well-known in the art.

According to a preferred embodiment of the present invention the mutation of at least one amino acid residue selected from the group consisting of X₃, X₉, X₁₂, X₁₇ and X₂₀ of SEQ ID No. 1 is a substitution or deletion.

The amino acid residues naturally occurring at the aforementioned positions within the motif of members of the wild-type polypeptide of the Niemann-Pick type C2 (NPC2) protein family can be substituted with other amino acid residues or deleted.

An "amino acid substitution", as used herein, refers to the replacement of at least one existing amino acid residue in a predetermined amino acid sequence with a second, different amino acid residue. An "amino acid deletion", as used herein, refers to the removal of at least one existing amino acid residue in a predetermined amino acid sequence.

Amino acid residues X₃, X₉, X₁₂, X₁₇ and X₂₀ of SEQ ID No. 1 may be substituted with another naturally occurring amino acid residue. Thereby, it is preferred to substitute amino acid residue X₃ of SEQ ID No. 1 with a small amino acid residue. X₉ of SEQ ID No. 1 is preferably substituted with a negatively charged amino acid residue. X₁₂ of SEQ ID No. 1 is preferably substituted with a polar and/or small and/or charged and/or negatively charged amino acid residue. X₁₇ of SEQ ID No. 1 is preferably substituted with a polar and/or charged and/or negatively charged amino acid residue. X₂₀ of SEQ ID No. 1 is preferably substituted with a polar and/or large amino acid residue. Polar amino acid residues include preferably ASP, GLU, ARG, LYS, HIS, ASN, GLN, SER, THR and TYR. Charged amino acid residues include preferably ARG, HIS, LYS, ASP and GLU. Negatively charged amino acid residues include preferably ASP and GLU. Small amino acid residues include preferably ASN, ASP, PRO, THR and VAL. Large amino acid residues include preferably ARG, GLN, GLU, HIS, ILE, LEU, LYS, MET, PHE, TRP and TYR.

According to a preferred embodiment of the present invention X₃ of the variant is T, P, C, Y, V, S, W, F, I, N, or G, preferably T, W, Y, P, C, V, S, I, N or G, more preferably T.

According to another preferred embodiment of the present invention X₉ of the variant is E, Q or D, preferably E.

According to a preferred embodiment of the present invention X₁₂ of the variant is D, N, E, P, S, Q, G or T, preferably D or E, more preferably D.

According to a further preferred embodiment of the present invention X₁₇ of the variant is E, D, N, Q, or G, preferably E or D, more preferably E.

According to a preferred embodiment of the present invention X₂₀ of the variant is A, Q, W, M, E, L, Y, N, T, S, I, F, D, or G, preferably Q.

The variant of the present invention may comprise further mutations within the motif consisting of SEQ ID No. 1. According to a preferred embodiment of the present invention X₁₄ and/or X₁₆ of SEQ ID No. 1 are mutated, preferably by substitution or deletion.

According to a preferred embodiment of the present invention X₁₄ of the variant is E, K or D, preferably E.

According to a further preferred embodiment of the present invention X₁₆ of the variant is D, E, K, or R, preferably E or D.

According to another preferred embodiment of the present invention the motif consists of an amino acid sequence which is at least 90% identical to an amino acid sequence selected from the group consisting of SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11 and SEQ ID No. 12.

The motif of polypeptide members of the Niemann-Pick type C2 (NPC2) protein family may comprise amino acid sequences which are at least 90%, preferably at least 91%, more preferably at least 92%, more preferably at least 93%, more preferably at least 94%, more preferably at least 95%, more preferably at least 96%, more preferably at least 97%, more preferably at least 98%, more preferably at least 99%, in particular 100%, identical to any one of SEQ ID Nos. 2 to 12.

The amino acid sequences SEQ ID Nos. 2 to 12 as well as the polypeptide (allergen) from which these sequences are derived from can be found in the following table:

| **SEQ ID No.** | **Amino acid sequence** | **Allergen** | **UniProt Acc. No.** |
|---|---|---|---|
| 2 | NQNSKTAKIEIKASIDGLEVDVPGIDPNAC HYMKCPLVKGQQYDIKYTWNVPKIAPK | Der p 2 | P49278, variant V57L, T64S, D131N (= Der p 2.0103) |
| 3 | NQDTASVGLVLTAIIDGDIAIDIP- GLETNACKLMKCPIRKGEHQELI- YNIGEIPDATPE | Gly d 2.01 | Q9U5P7 |
| 4 | NQDTAKVTIKVLAKVAG- TTIQVPGLETDGCKFIKCPVKKGEALD- FIYSGTIPAITPK | Lep d 2 | P80384 |
| 5 | NQDSPTAVIKISAKVNGVELQVPGI- ETNGCHHMKCPLVKGQSYQFKYDLVIPQIL PN | Sui m 2 | Q2TUH5 |
| 6 | NQDSEKLEIKISAIMN- NIEVPVPGVDKDGCK- HTTCPLKKGQKYELDYSLIIPTILPN | Blo t 2 | A6XEN9 |
| 7 | NQDSAKIEVHL- TANIDGLSIPIPGVDKDGCKYVTCPIK- KGEKVHFNYSLVVPKLIPN | Ale o 2 | Q2VXY3 |
| 8 | NQDTAKISVHLSAKVEGLEIP- IPGVDRDGCKYVKCPVKKGEHLHLNYAL- TVPKLLPN | Tyr p 2 | A7XZL0 |
| 9 | NQDTAHVKIQLTASMG- GIEIPVPGVGTDGCKY- IKCPLVKGEKKTFPYTYLVPKILPS | Aca s 2 | A7XZH1 |
| 10 | NQDSANLKLDIVAD- INGVQIEVPGVDHDGCHYVKCPIK- | Pso o 2 | Q965E2 |
| | KGQHFDVKY TY SIP AILP T | | |
| 11 | NQNTKTAKIEIKASLD- GLEIDVPGIDTNACH- FMKCPLVKGQQYDIKYTWNVPKIAPK | Der f 2 | Q00855 |
| 12 | NQNSNAAKIEIKATIDGVEIDVPGID- NNLCHFMKCPLVKGQEYDIKYTWNVPRI- APK | Eur m 2 | Q9TZZ2 |

The amino acid residues in bold, italic and underlined correspond to X₃, X₉, X₁₂, X₁₇ and X₂₀, respectively, of SEQ ID No. 1 and may thus be mutated to obtain a variant of the present invention. X₁₄ and/or X₁₆ can also mutated and are indicated as italic and underlined letters.

The terms "identity" and "identical", as used herein, indicates whether any two (or more) amino acid or nucleic acid sequences are "identical" to a certain degree ("% identity") to each other. This degree can be determined using known computer. Sequence identity is preferably determined by BLAST alignment (http://blast.ncbi.nlm.nih.gov/; Altschul SF et al J. Mol. Biol. 215(1990):403-410) using the BLOSUM62 matrix, a gap existence penalty of 11, and a gap extension penalty of 1.

According to a preferred embodiment of the present invention the polypeptide of the Niemann-Pick type C2 (NPC2) protein family comprises a second motif consisting of the amino acid sequence X₂₁HX₂₂GX₂₃X₂₄X₂₅X₂₆X₂₇ (SEQ ID No. 13), wherein X₂₁ is L or I, X₂₂ is R or K, X₂₃ is K, T or E, X₂₄ is K, P, A, E or S, X₂₅ is F, L or M, X₂₆ is Q, K, A, T, S or D and X₂₇ is L or M.

The polypeptide of the Niemann-Pick type C2 (NPC2) protein family to be modified according to the present invention to obtain a variant may comprise a second motif. Said second motif consists of amino acid sequence SEQ ID No. 13 and is typically located in between the N-terminal end of the polypeptide of the Niemann-Pick type C2 (NPC2) protein family and the N-terminal end of the motif having amino acid sequence SEQ ID No. 1.

According to a preferred embodiment of the present invention X₂₆ of SEQ ID No. 13 is mutated by substitution or deletion. If X₂₆ of SEQ ID No. 13 is substituted with another amino acid residue X₂₆ of the variant is preferably H, K, R, D, or E, preferably K.

According to another preferred embodiment of the present invention the second motif consists of an amino acid sequence which is at least 80% identical to an amino acid sequence selected from the group consisting of SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23 and SEQ ID No. 24.

The second motif of polypeptide members of the Niemann-Pick type C2 (NPC2) protein family may comprise amino acid sequences which are at least 80%, preferably at least 85%, in particular 100%, identical to any one of SEQ ID Nos. 14 to 24.

The amino acid sequences SEQ ID Nos. 14 to 24 as well as the polypeptide (allergen) from which these sequences are derived from can be found in the following table:

| **SEQ ID No.** | **Amino acid sequence** | **Allergen** | **UniProt Acc. No.** |
|---|---|---|---|
| 14 | HRGKPF***Q***L | Der p 2 | P49278, variant V57L, T64S, D131N, (= Der p 2.0103) |
| 15 | HRGKPLTL | Gly d 2.01 | Q9U5P7 |
| 16 | HRGEKMTL | Lep d 2 | P80384 |
| 17 | HKGKKL***S***M | Sui m 2 | Q2TUH5 |
| 18 | HKGKSFTL | Blo t 2 | A6XEN9 |
| 19 | HKGKEL***K***L | Ale o 2 | Q2VXY3 |
| 20 | HKGKPLAL | Tyr p 2 | A7XZL0 |
| 21 | HKGKEL***K***L | Aca s 2 | A7XZH1 |
| 22 | HKGKKL***D***L | Pso o 2 | Q965E2 |
| 23 | HRGKPF***T***L | Der f 2 | Q00855 |
| 24 | HRGTAF***Q***L | Eur m 2 | Q9TZZ2 |

The amino acid residue in bold, italic and underlined corresponds to X₂₆ of SEQ ID No. 13 and may thus be mutated to obtain a variant of the present invention.

According to another preferred embodiment of the present invention the polypeptide of the Niemann-Pick type C2 (NPC2) protein family comprises an amino acid sequence selected from the group consisting of SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34 and SEQ ID No. 35.

The polypeptide of the Niemann-Pick type C2 (NPC2) protein family which is the basis of the variant of the present invention may comprise amino acid sequences which are at least 90%, preferably at least 91%, more preferably at least 92%, more preferably at least 93%, more preferably at least 94%, more preferably at least 95%, more preferably at least 96%, more preferably at least 97%, more preferably at least 98%, more preferably at least 99%, in particular 100%, identical to any one of SEQ ID Nos. 25 to 35.

The amino acid sequences SEQ ID Nos. 25 to 35 as well as the polypeptide (allergen) from which these sequences are derived from can be found in the following table:

| **SEQ ID No.** | **Amino acid sequence** | **Allergen** | **UniProt Acc. No.** |
|---|---|---|---|
| 25 | DQVDVKDCANHEIKKVLVPGCHGSEP- CIIHRGKPFQLEALFE- ANQNSKTAKIEIKASIDGLEVDVPGIDPNACHY MKCPLVKGQQYDIKYTWNVPKIAPKSEN- VVVTVKVMGDNGVLACAIATHAKIRD | Der p 2 | P49278, variant V57L, T64S, D131N, (= Der p 2.0103) |
| 26 | GKMNFTDCGHNEIKELSVSNCTGNYCVIHRGK- PLTLDAKFDANQDTASVGLVLTAIIDGDIAI- | Gly d 2.01 | Q9U5P7 |
| | DIPGLETNACKLMKCPIRKGEHQELI- YNIGEIPDATPEIKAKVKAQLIGEHGVLAC- GWVDGEVQE | | |
| 27 | GKMTFKDCGHGEV- TELDITGCSGDTCVIHRGEKMTLEAK- FAANQDTAKVTIKVLAKVAG- TTIQVPGLETDGCKFIKCPVKKGEALD- FIYSGTIPAITPKVKADVTAELIGDHGVMAC- GTVHGQVE | Lep d 2 | P80384 |
| 28 | GEMKFQDCGHGEV- KKLLVSDCSGDYCIIHKGKKLSMEADFVANQD- SPTAVIKISAKVNGVELQVPGI- ETNGCHHMKCPLVKGQSYQFKYDLVIPQILPNV KADVTASLTGAHGLLACGTVHGEVQN | Sui m 2 | Q2TUH5 |
| 29 | RCQIYRSCTWEVTSLDLSGCSGDHCIIHKGKS- FTLKTFFIANQDSEKLEIKISAIMN- NIEVPVPGVDKDGCKHTTCPLKKGQKYELDYS- LIIPTILPNLKTVTTASLVGDHGVVACGKVN- TEVVD | Blo t 2 | A6XEN9 |
| 30 | GQVRFEDCGHHEVTKLDIS- QCADGASTCVIHKGKELKLDAEILANQD- SAKIEVHL- TANIDGLSIPIPGVDKDGCKYVTCPIK- KGEKVHFNYSLVVPKLIPNLHN- VEVGAKITGDHGLLACLRLKGDIAN | Ale o 2 | Q2VXY3 |
| 31 | GNVKFEDCGHHEVVKLNISQCADGVATCVLHKG KPLALDAEMISNQDTAKISVHLSAKVEGLEIP- IPGVDRDGCKYVKCPVKKGEHLHLNYAL- TVPKLLPNLHNVEIGAKISGDHGLLA- CLRLHGDLSN | Tyr p 2 | A7XZL0 |
| 32 | TPIPYTDCGNHELSKLDANLHCAGDLCTI- HKGKELKLDAQFTVNQDTAHVKIQLTASMG- GIEIPVPGVGTDGCKY- IKCPLVKGEKKTFPYTYLVPKILPSVTGDV- KAVFTGDHGPLGCLKFHGKIVD | Aca s 2 | A7XZH1 |
| 33 | GKVKFQDCGKGEVESLEVEGCSGDYCVIHKGKK LDLAISVTSNQDSANLKLDIVAD- INGVQIEVPGVDHDGCHYVKCPIK- KGQHFDVKYTYSIPAILPTTKAKIIAKII- GDKGLGGCIVINGEIQD | Pso o 2 | Q965E2 |
| 34 | DQVDVKDCANNEIKKVMVDGCHGSDPCIIHRGK PFTLEALFDANQNTKTAKIEIKASLD- GLEIDVPGIDTNACH- FMKCPLVKGQQYDIKYTWNVPKIAPKSEN- VVVTVKLIGDNGVLACAIATHGKIRD | Der f 2 | Q00855 |
| 35 | DQVDIKDCANHEIKKVMVPGCK- GSEPCVIHRGTAFQLEAVFDANQNSNAAKIEIK ATIDGVEIDVPGIDNNLCH- FMKCPLVKGQEYDIKYTWNVPRIAPKSEN- VVVTVKLLGDNGVLACAIATHAKIRD | Eur m 2 | Q9TZZ2 |

The amino acid residues in bold, italic and underlined correspond to X₃, X₉, X₁₂, X₁₄, X₁₆, X₁₇, X₂₀ and X₂₆ respectively, of SEQ ID No. 1 and may thus be mutated to obtain a variant of the present invention.

According to a particular preferred embodiment of the present invention the variant of the present invention are derived from Der p 2 and Blo t 2 (Uniprot ID A6XEN9). Preferred variants may thus comprise or consist of SEQ ID No. 36 (Der p 2 derivative) or SEQ ID No. 37 (Blo t 2 (Uniprot ID A6XEN9) derivative).
SEQ ID No. 36: 
DQVDVKDCANHEIKKVLVPGCHGSEPCIIHRGKPFKLEALFEANQNSTTAKIEIKASIDGLEVD
VPGIDPNACEYMKCPLVDGQQYDIKYEWDVPEIAPQSENVVVTVKVMGDNGVLACAIATHAKIR
D
SEQ ID No. 37:

GDVKFTDCAHGEVTSLDLSGCSGDHCTIHKGKSFTLKTFFIANQDSEKLEIKISATMNGIEVPV
PGVDKDGCKHTTCPLKKGQKYELDYSLIIPTVLPNVKTVTTASLVGDHGVVACGKVNTEVVD

In order to stabilise the variants of the present invention (for instance during recombinant expression) the N-terminal amino acid residues, preferably the first two residues or at least the second, are preferably exchanged by respective stabilising amino acid residues (e.g. glycine or glycine-alanine) (see e.g. Varshavsky A. Nature Structural & Molecular Biology 15(2008):1238-40 ; Dougan DA et al. Molecular Microbiology 76(2010): 545-558 ; Tobias JW, et al. Science 254(1991):1374-7). Alternatively, it is also possible to bind to the N-terminal end of the variants of the present invention additional amino acid residues or di- or tripeptides such as glycine or glycine-alanine.

One purpose to modify polypeptides of the Niemann-Pick type C2 (NPC2) protein family as disclosed herein, in particular allergens of this patent family, is to obtain variants or polypeptide variants exhibiting a reduced allergenic activity compared to the corresponding wild-type polypeptide of the Niemann-Pick type C2 (NPC2) protein family comprising SEQ ID No. 1. The allergenic activity of a molecule (i.e. allergen) is usually a measure of its capability to bind IgE molecules. Therefore, the allergenic activity of a molecule can be determined by measuring its IgE binding capacity or IgE reactivity. The IgE reactivity in the variant of the present invention is - compared to the corresponding wild-type polypeptide - preferably reduced. Using an antibody (IgE)-based biologic assay (e.g. inhibition ELISA or RAST, basophil inflammatory mediator release), at least 10, preferably at least 50, more preferably at least 100, even more preferred at least 200 fold higher concentrations of the variant are required to reach half-maximal values obtained with wild type polypeptide as defined herein.

Variants of allergens showing a reduced IgE binding capability are usually called "hypoallergenic". Therefore, the variants of the present invention are preferably hypoallergenic variants or hypoallergenic polypeptides. The term "hypoallergenic" refers to the ability of polypeptide derived from an allergen or another hypoallergenic molecule with allergenic properties to exhibit reduced or no allergic IgE-mediated reactions when administered to an individual. The reduced or missing ability of "hypoallergenic" derivatives of an allergen to induce an allergic reaction in an individual is a result of the removal or destruction of IgE binding epitopes from said allergens or of the fact that the IgE binding epitopes are no longer sterically accessible to their corresponding IgE molecules. In an embodiment of the present invention a hypoallergenic polypeptide (i.e. variant of the present invention) exhibits an IgE reactivity or binding which is at least 10%, at least 20 %, at least 30 %, at least 50 %, at least 60 %, at least 70 %, at least 80 %, at least 90 %, at least 95 %, at least 98 % or at least 99 % lower than the IgE reactivity of the allergen or fragment thereof or the hypoallergenic variant of said allergen or fragment. IgE reactivity of molecules can be routinely tested by IgE western blots using pools of sera of patients comprising IgE molecules binding specifically to the molecule of interest. Reduction of IgE binding of the respective hypoallergenic molecule will be assessed by comparing it to the IgE binding of the wild-type or precursor molecule or the molecule from which the protein, polypeptide and peptide are derived from. In the US 2009/0104208 (see e.g. example 2), for instance, methods for determining the IgE reactivity are described. A molecule exhibiting a lower IgE reactivity as the molecule from which it is derived from is to be considered hypoallergenic.

Due to the reduced IgE binding capacity of the variants of the present invention and since all or substantially all T cell epitopes of the polypeptide are conserved in the variant of the present invention, these molecules can be used in the treatment and/or prevention of allergies. Therefore, a preferred embodiment of the present invention relates to the use of a variant of the present invention in the treatment or prevention of allergies caused by polypeptide of the Niemann-Pick type C2 (NPC2) protein family. The type of allergy to be treated or prevented depends on the allergen used to obtain the variant of the present invention. Since some allergens show cross-reactivity to other allergens, the treatment or prevention can be expanded to other allergies. Cross-reactivities between various allergens are well known to a person skilled in the art.

Another aspect of the present invention relates to a nucleic acid molecule encoding a variant of the present invention. The nucleic acid molecule may be a DNA or RNA molecule.

A further aspect of the present invention relates to a vector comprising a nucleic acid molecule of the present invention.

Another aspect of the present invention relates to host cell comprising a nucleic acid molecule or a vector of the present invention.

The nucleic acid molecule of the present invention encoding the variant of the present invention may be operably linked to transcriptional and translational control sequences, e.g. promoters or enhancers, to direct or modulate RNA synthesis and expression. The expression control sequence can be in an expression vector. Exemplary bacterial promoters include lad, lacZ, T3, T7, gpt, lambda PR, PL and trp. Exemplary eukaryotic promoters include CMV immediate early, HSV thymidine kinase, early and late SV40, LTRs from retrovirus, and mouse metallothionein I.

Promoters suitable for expressing, or over-expressing, a polypeptide in bacteria include the *E. coli* lac or trp promoters, the lad promoter, the lacZ promoter, the T3 promoter, the T7 promoter, the gpt promoter, the lambda PR promoter, the lambda PL promoter, promoters from operons encoding glycolytic enzymes such as 3-phosphoglycerate kinase (PGK), and the acid phosphatase promoter. Eukaryotic promoters include the CMV immediate early promoter, the HSV thymidine kinase promoter, heat shock promoters, the early and late SV40 promoter, LTRs from retroviruses, and the mouse metallothionein-I promoter. Other promoters known to control expression of genes in prokaryotic or eukaryotic cells or their viruses may also be used. The present invention relates also to expression systems such as expression cassettes, vectors, cloning vehicles and the like, comprising nucleic acids of the invention, e.g., sequences encoding the variants of the invention, for expression, and over-expression, of the polypeptides of the invention. Expression vectors and cloning vehicles of the invention can comprise viral particles, baculovirus, phage, plasmids, phagemids, cosmids, fosmids, bacterial artificial chromosomes, viral DNA (e.g., vaccinia, adenovirus, foul pox virus, pseudorabies and derivatives of SV40), Pl - based artificial chromosomes, yeast plasmids, yeast artificial chromosomes, and any other vectors specific for specific hosts of interest (such as bacillus, Aspergillus and yeast). Exemplary bacterial vectors include pQE vectors (Qiagen), pBluescript plasmids, pNH vectors, (lambda-ZAP vectors (Stratagene); ptrc99a, pKK223-3, pDR540, pRIT2T (Pharmacia); Eukaryotic: pXTl, pSG5 (Stratagene), pSVK3, pBPV, pMSG, pSVLSV40 (Pharmacia). However, any other plasmid or other vector may be used so long as they are replicable and viable in the host. Low copy number or high copy number vectors may be employed with the present invention.

As representative examples of expression vectors which may be used there may be mentioned viral particles, baculovirus, phage, plasmids, phagemids, cosmids, fosmids, bacterial artificial chromosomes, viral DNA (e.g., vaccinia, adenovirus, foul pox virus, pseudorabies and derivatives of SV40), Pl -based artificial chromosomes, yeast plasmids, yeast artificial chromosomes, and any other vectors specific for specific hosts of interest (such as bacillus, Aspergillus and yeast). Thus, for example, the DNA may be included in any one of a variety of expression vectors for expressing a polypeptide. Such vectors include chromosomal, nonchromosomal and synthetic DNA sequences. Large numbers of suitable vectors are known to those of skill in the art.

The vector of the present invention may be introduced into the host cells using any of a variety of techniques, including transformation, transfection, transduction, viral infection, gene guns, or Ti-mediated gene transfer.

The host cell of the present invention may be any of the host cells familiar to those skilled in the art, including prokaryotic cells, eukaryotic cells, such as bacterial cells, fungal cells, yeast cells, mammalian cells, insect cells, or plant cells. Exemplary bacterial cells include any species within the genera *Escherichia, Bacillus, Streptomyces, Salmonella, Pseudomonas, Lactococcus, and Staphylococcus, including,* e.g., *Escherichia coli, Lactococcus lactis, Bacillus subtilis, Bacillus cereus, Salmonella typhimurium, Pseudomonas fluorescens.* Exemplary fungal cells include any species of *Aspergillus,* including As*pergillus niger.* Exemplary yeast cells include any species of *Pichia, Saccharomyces, Schizosacchammyces,* or *Schwanniomyces, including Pichia pastoris, Saccharomyces cerevisiae, or Schizosaccharomyces pombe.* Exemplary insect cells include any species of *Spodoptera* or *Drosophila,* including *Drosophila S2* and *Spodoptera S*/*P.* Exemplary insect cells include *Drosophila S2* and *Spodoptera Sf9.* Exemplary animal cells include CHO, COS or Bowes melanoma or any mouse or human cell line. The selection of an appropriate host is within the abilities of those skilled in the art.

Another aspect of the present invention relates to a pharmaceutical preparation comprising a variant according to the present invention.

According to a preferred embodiment of the present invention said preparation comprises further at least one pharmaceutically acceptable excipient, diluent, adjuvant and/or carrier.

In order to provide a vaccine formulation which can be administrated subcutaneously, intramuscularly, intravenously, mucosally etc. to an individual the formulation has to comprise further excipients, diluents, adjuvants and/or carriers. Preferred adjuvants used comprise KLH (keyhole-limpet hemocyanin) and alum. Suitable protocols for the production of vaccine formulations are known to the person skilled in the art and can be found e.g. in "Vaccine Protocols" (A. Robinson, M.P. Cranage, M. Hudson; Humana Press Inc.,U.S.; 2nd edition 2003).

The present invention is further illustrated by the following figures and examples without being restricted thereto.
Fig. 1 shows a sequence alignment of Der p 2.0103 and Der p 2-3x. The mutated amino acid residues located within the B cell epitopes (Q38, T93 and N95 of Der p 2.0103; see Szalai et al., Mol Immunol. 2008 Mar;45(5):1308-17.) are shown underlined and bold).
Fig. 2 showsimmunoblots with patients' sera and isolated naturally occurring Der p 2 ("nDer p 2"), recombinantly produced wild-type Der p 2 ("rDer p 2 WT"), codon harmonized Der p 2 ("rDer p 2 WT harmonized") and mutant protein Der p 2-3x in bacterial lysate.
Fig. 3 shows the amino acid sequence of Der p 2-8x whereby the N-terminal GA peptide and the mutated amino acid residues are marked with a frame.
Fig. 4 shows a sequence alignment between Der p 2.0103 and Der p 2-8x whereby point mutations introduced into the B cell epitope (Szalai et al., Mol Immunol. 2008 Mar;45(5):1308-17.) are underscored and point mutations of amino acid residues on the surface are underscored, bold and italic.
Fig. 5 shows isolated naturally occurring Der p 2 and wild-type Der p 2 and Der p 2-8x recombinantly produced in *E. coli* on an SDS PAGE gel after their purification from the soluble fraction by ion exchange chromatography.
Fig. 6 shows circular dichroism (CD) measurements of rDer p 2-8x in comparison to the wildtype molecule rDer p 2 at 20°C and 95°C.
Fig. 7 shows FTIR measurements of rDer p 2-8x in comparison to the wildtype molecule rDer p 2.
Fig. 8 shows proteolytic stability of rDer p 2-8x in comparison to the wildtype molecule rDer p 2 by using the lysosomal fraction of JAWS II cells.
Fig. 9 shows the results of ANS displacement assays using rDer p 2-8x and wildtype molecule rDer p 2.
Fig. 10 shows reduced IgE binding of rDer p 2-8x in comparison to its recombinantly produced parental allergen (Fig. 10A) as determined by ELISA. The same results were obtained in mediator release assays, where rDer p 2-8x in contrast to recombinantly produced rDer p 2 showed a 244-fold reduction in the IgE binding capacity (Fig. 10B), as determined by comparing the mean amount of protein for each antigen, necessary to trigger half maximal mediator release.
Fig. 11 shows that rDer p 2-8x is able to induce IgG1 as well as IgG2a antibodies cross-reactive with the wild-type allergen Der p 2. Animals immunized with rDer p 2 are represented with white, animals immunized with rDer p 2-8x in grey boxes; pre-sera are represented with stripes.
Fig. 12 shows the results of an ELISpot assay used to determine the cytokine levels secreted from splenocytes. Animals immunized with rDer p 2 are represented with white, animals immunized with rDer p 2-8x in grey boxes.
Fig. 13 shows the amino acid sequence of Blo t 2.2-8x.
Fig. 14 shows a sequence alignment of Blo t 2.2 with the mutant Blo t 2.2-8x whereby the greyed amino acid residue positions are the mutation sites.
Fig. 15 shows an immunoblot with pool of patients' sera (n= 10). The lanes were loaded with 0.5 µg total protein as follows: lane 1 purified Blo t 2.2, lane 2 *E. coli* extract comprising Blo t 2.2, lane 3 *E. coli* extract comprising Blo t 2.2-8x, lane 4 E. *coli* extract, lane 5 recombinantly produced wild-type Der p 2 and lane 6 recombinantly produced Der p 2-8x.
Fig. 16 shows the results of an IgE ELISA with patients' sera (n= 10) using ELISA plates coated with purified Blo t 2.2, *E. coli* extract comprising Blo t 2.2, *E. coli* extract comprising Blo t 2.2-8x and *E. coli* extract. For the statistical analysis paired samples t-test was used.

### EXAMPLES:

### Example 1: Der p 2 variants comprising three point mutations at Q38, T93 and N95

In the present example a novel low IgE-binding variant of the mite allergen Der p 2 was investigated as a possible future candidate for house dust mite allergen-specific immunotherapy (HDM AIT). Therefore, the structure of Der p 2 and the surface charge of the allergen were analyzed. Thereafter, amino acids were identified and changed in a way that the overall structure remained intact; however, the surface charged was changed radically.

The allergen Der p 2 from *Dermatophagoides pteronyssinus,* for instance, is classified as member of the group 2 allergens. Der p 2 is a small, about 14 kDa, globular protein which consist of 8 beta-sheets and a small alpha-helical structure (Derewenda U, J Mol Biol. 2002 Apr 19;318(1):189-97). As the structure was resolved, it has been revealed, that Der p 2 is a structural homologue to the human MD- (myeloid differentiation factor) 2 protein and therefore is able to substitute the function of MD-2 at the TLR4 receptor, thus leading to a TH2 immune response.

First a model, named Der p 2 Mod was generated by utilizing MODELLER (Sali A and Blunell TL, J Mol Biol 234(1993):779-815), using the sequence of Der p 2 isoform 2.0103 (P49278, variant V57L, T64S, D131N) with a Gly-Ala tag added at the N-terminus of the protein.

Within the B cell epitope of Der p 2 (Der p 2 isoform 2.0103; Uniprot ID P49278, variant V57L, T64S, D131N) comprising amino acid residues 35 to 39 and 91 to 96 three amino acid residues at positions Q38, T93 and N95 have been mutated by exchanging them with K, E or E or D, respectively, thus obtaining a Q38K, T93E, N95E variant (Der p 2 Mod 1) and a Q38K, T93E, N95D variant (Der p 2 Mod 2). Due to these amino acid mutations polar amino acid residues were exchanged with polar or charged amino acid residues within the B-cell epitopes. In all models the Gly-Ala tag was added at the N-terminus of the polypeptide. The tag was applied to increase the protein expression in *E. coli.*

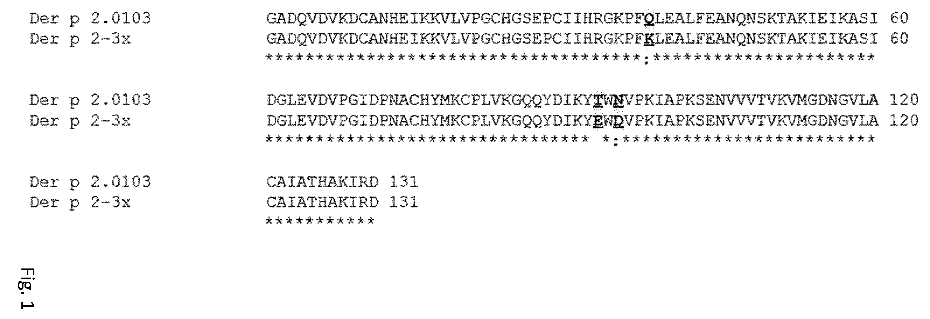

The aim of this example was to design a hypoallergenic variant of the group 2 allergen from *Dermatophagoides pteronyssinus* by *in silico* mutagenesis and test its properties in wet lab. Therefore, polar or charged amino acids which exist in the previously published B cell epitope (Szalai et al., Mol Immunol. 2008 Mar;45(5):1308-17) were exchanged to either charged or polar amino acids. As the overall structure of Der p 2, in particular its three-dimensional structure, should not be altered, the variant was, after molecular modelling, evaluated by its energetic stability regarding the Z-score by using the program Pro-SA-2003 (Sippl M.J, Proteins 17:355-362, 1993).
This software uses knowledge-based potentials derived from known protein structures and captures the average properties of native globular proteins in terms of atom-pair and solvent interactions to generate scores reflecting the quality of protein structures (Sippl M.J, Proteins 17:355-362, 1993). The calculations revealed that Z-scores were -8.41 (Der p 2 Mod 1) and -8.39 (Der p 2 Mod 2). These data indicated that the structural models of Der p 2 Mod 1 and 2 were energetically stable. The variant (Q38K, T93E, N95D (Der p 2 Mod 2; "Der p 2-3x") was tested experimentally. Including the Gly-Ala expression tag, the resulting molecule Der p 2 Mod 2 (Z-score: -8.39) scored better than the wildtype polypeptide Der p 2 Mod (-8.17) by 0.22 arbitrary units, indicating a stable mutant polypeptide. Mutation points on the B cell epitope are depicted in fig. 1B.

Based on the model Der p 2 Mod, the protein Der p 2.0103 with an N-terminal GA tag, termed Der p 2.0103 thereafter, was generated. Based on the model Der p 2 Mod 2, the protein Der p 2-3x with an N-terminal GA tag, termed Der p 2-3x thereafter, was generated. A sequence alignment between Der p 2.0103 and Der p 2-3x is shown in Fig. 1.

In order to determine the presence of IgE epitopes on the mutated Der p 2 variants sera of house dust mite allergic patients who were sensitized to Der p 2 from *Dermatophagoides pteronyssinus* were used. The IgE-reactivity to Der p 2 was determined by ELISA and immunoblot analysis (Gadermaier G. et al., J Allergy Clin Immunol 134(2014):472-5).

Der p 2 (Uniprot ID P49278, variant V57L, T64S, D131N + N-terminal GA) and Der p 2-3x + N-terminal GA were codon harmonized (Angov E, et al. PloSOne 3(2008):e2189) and thereafter genes were ordered from ATG Biosynthetics, Merzhausen, Germany. All genes were cloned in a pET28b vector using the restriction sites NcoI and EcoRI. Expression plasmids were transformed in E. *coli* Shuffle (New England Biolabs, Ipswich, MA, USA) and grown at 37°C to an OD600 of 0.6 in LB medium supplemented with 10 mL/L glycerol, 2 mM MgSO₄, 2 g/L ammonium sulfate, 10 mM NaP-buffer, pH 7.4, 25 mg/L kanamycin. The protein production was induced by adding 0.5 mM isopropyl-β-D-thiogalactopyranoside (Applichem) and the culture was incubated for 3 hours at 37°C. After the cells were harvested by low speed centrifugation, the bacterial cell pellet was resuspended in appropriate lysis buffer.

*E. coli* lysates and purified protein was analyzed by denaturing sodium dodecyl polyacrylamide gel electrophoresis (SDS-PAGE) using 15% gels. Proteins were visualized by staining with Coomassie Brilliant Blue R-250 (Biorad). For immunoblot analysis proteins were separated by SDS-PAGE and subsequently blotted on nitrocellulose membranes (GE healthcare, UK). Patients' sera were added in a 1:20 dilution in blocking buffer and incubated at 4°C over night. For the detection of bound IgE, an alkaline phosphatase conjugated mouse anti-human IgE antibody (BD Pharmingen, San Diego, CA, USA) was added. Nitro blue tetrazolium chloride (NBT)/ 5-bromo-4 chloro-3-indolyl phosphate (BCIP) (both Applichem) in buffer was added for the colorimetric reaction.

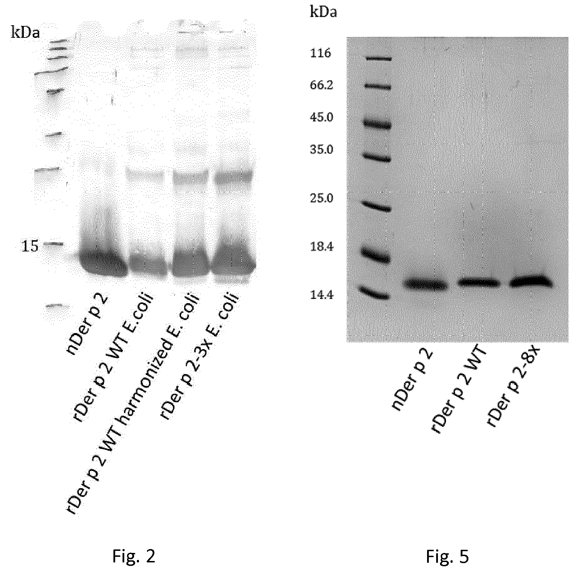

After the polypeptide Der p 2-3x was expressed in *E. coli* Shuffle strain (New England Biolabs, Ipswich, MA, USA), immunoblots with patients' sera and mutant protein Der p 2-3x, also expressed in *E. coli* Shuffle, were performed using bacterial lysates (Fig. 2). The results showed no reduced IgE-binding capacity compared to two wildtype Der p 2 polypeptides recombinantly produced.

### Example 2: Der p 2 variants comprising up to eight point mutations at Q38, K50, H76, K84 T93, N95, K98, and K102 of wildtype Der p 2

*Design of the point mutations in Der p 2 and its homologs modeling the 3D structure Der p 2 Mod.*

First a model, named Der p 2 Mod was generated by utilizing MODELLER (Sali A and Blunell TL, J Mol Biol 234(1993):779-815), using the sequence of Der p 2 isoform 2.0103 (P49278, variant V57L, T64S, D131N) with a Gly-Ala tag added at the N-terminus of the protein. The tag was applied to increase the protein expression in *E. coli.* As template for the computer model the PDB entry 1KTJ was used. Thereafter, both proteins 1KTJ and Der p 2 Mod were evaluated with the ProSA-2003 (Sippl M.J, Proteins 17:355-362, 1993) resulting in Z-scores of -8.04 and -8.17, respectively.

### Mutation experiment based on Der p 2 Mod

The following set of rules was applied for the design of the Der p 2-8x molecule comprising mutation within amino acid residues Q38, K50, H76, K84 T93, N95, K98, and K102:
a) By using a ProSA-2003 (Sippl M.J, Proteins 17:355-362, 1993)based greedy search, iteratively introduce mutations in the known B-cell epitopes (amino acid position regarding Der p 2 Mod: 35-39, 91-96; see example 1). Exchanges from polar to polar amino acids (including charged ones) within the B-cell epitope have been performed. As the change in side chain size is smaller for a N->D substitution than a N->E exchange, mutations (Q38K, T93E, N95D; "Der p 2-3x") were selected for the remaining procedure and the B-cell epitope are excluded from further exchanges.
b) Outside the B-cell epitope all exposed and charged amino acid residues were selected as potential mutation sites. Exposed residues had a relative solvent accessible area larger than 60%, using the method of the Protein Geometry library (Voss NR and Gerstein M, J Mol Biol. 2005 Feb 18;346(2):477-92) with a probe sphere size of 1.4 Å.
c) With molecular graphics, the mutation sites were further restricted to the front side of Der p 2 Mod, leaving the back side untouched for potential IgG antibody binding. The front side of the molecule is defined as the side of the beta-sheet, which contains the known B-cell epitope.
d) Then, iteratively mutations in the remaining sites using a ProSA-2003 (Sippl M.J, Proteins 17:355-362, 1993)based greedy search were introduced. The Z-score improved by exchanging up to 8 amino acid residues. If more than 8 amino acid residues were exchanged the Z-score did not improve further.
e) From equivalently scoring mutations, (i) mutants from or to Arg and Lys were preferred. (ii) Also, positions conserved in the known Der p 2 isoforms and homologous proteins were favored. (iii) Finally mutations where the change in the size of the side chains is larger were preferred, as this has a larger influence on the surface shape.

The Der p 2 variants listed in Table 1 have been designed.

**Table 1:**

| | **Der p 2 variant - Mutations** | **Z-score** |
|---|---|---|
| | Q38K,T93E,N95D,K50T,H76E,K98E,K102Q,K79E | -9.00 |
| **Der p 2-8x** | Q38K,T93E,N95D,K50T,H76E,K98E,K102Q,K84D | -8.98 |
| | Q38K,T93E,N95D,K50T,H76E,K98E,K102Q,K84N | -8.98 |
| | Q38K,T93E,N95D,K50T,H76E,K98E,K102Q,K79D | -8.97 |
| | Q38K,T93E,N95D,K50T,H76E,K98E,K102Q,K84E | -8.97 |
| | Q38K,T93E,N95D,K50T,H76E,K98E,K102Q,H13R | -8.96 |
| | Q38K,T93E,N95E,H76E,K50T,K98E,K102Q,K79E | -9.03 |
| | Q38K,T93E,N95E,H76E,K50T,K98E,K102Q,K84D | -9.01 |
| | Q38K,T93E,N95E,H76E,K50T,K98E,K102Q,K84N | -9.01 |
| | Q38K,T93E,N95E,H76E,K50T,K98E,K102Q,K79D | -9.00 |
| | Q38K,T93E,N95E,H76E,K50T,K98E,K102Q,H13R | -8.99 |
| | Q38K,T93E,N95E,H76E,K50T,K98E,K102Q,K84E | -8.99 |
| | Q38K,T93E,N95E,H76E,K50T,K98E,K102Q,D131 P | -8.99 |
| | Q38K,T93E,N95E,H76E,K50T,K98E,K102Q,K84P | -8.98 |
| | Q38K,T93E,N95E,H76E,K50T,K98E,K102Q,H13W | -8.98 |
| | Q38K,T93E,N95E,H76E,K50T,K98E,K102Q,K84S | -8.97 |

### Patients' sera

House dust mite allergic patients who were sensitized to Der p 2 from *Dermatophagoides pteronyssinus* were selected based on typical case history. Moreover, IgE-reactivity to Der p 2 was verified by ELISA and immunoblot analysis.

### Expression and purification of Der p 2-8x

Der p 2-8x + N-terminal GA was codon harmonized (Angov E, et al. PloSOne 3(2008):e2189) and thereafter the gene was ordered from ATG Biosynthetics, Merzhausen, Germany. The gene cloned in a pET28b vector using the restriction sites NcoI and EcoRI. Expression plasmids were transformed in *E. coli* Shuffle (New England Biolabs, Ipswich, MA, USA) and grown at 37°C to an OD600 of 0.6 in LB medium supplemented with 10 mL/L glycerol, 2 mM MgSO₄, 2 g/L ammonium sulfate, 10 mM NaP-buffer, pH 7.4, 25 mg/L kanamycin. The protein production was induced by adding 0.5 mM isopropyl-β-D-thiogalactopyranoside (Applichem, Darmstadt, Germany) and the culture was incubated for 3 hours at 37°C. After the cells were harvested by low speed centrifugation, the bacterial cell pellet was resuspended in appropriate lysis buffer. Der p 2-8x was purified from the soluble fraction by precipitation and ion exchange chromatography. Purified recombinant proteins were dialyzed against 10 mM sodium phosphate buffer, pH 7.4 (Applichem, Darmstadt, Germany) and stored at -20°C.

### SDS-PAGE and immunoblots

*E. coli* lysates and purified protein was analyzed by denaturing sodium dodecyl polyacrylamide gel electrophoresis (SDS-PAGE) using 15% gels. Proteins were visualized by staining with Coomassie Brilliant Blue R-250 (Biorad, Hercules, CA, USA). For immunoblot analysis proteins were separated by SDS-PAGE and subsequently blotted on nitrocellulose membranes (GE healthcare, Whatmann, UK). Patients' sera were added in a 1:20 dilution in blocking buffer and incubated at 4°C over night. For the detection of bound IgE, an alkaline phosphatase conjugated mouse anti-human IgE antibody (BD Pharmingen, San Diego, CA, USA) was added. Nitro blue tetrazolium chloride (NBT)/ 5-bromo-4 chloro-3-indolyl phosphate (BCIP) (both Applichem, Darmstadt, Germany) in buffer was added for the colorimetric reaction.

### Circular dichroism

Circular dichroism (CD) spectra of rDer p 2 wildtype and rDer p 2-8x were recorded in 10 mM sodium phosphate buffer pH 7.4 with a JASCO J-815 spectropolarimeter (Jasco, Tokyo, Japan) fitted with a Jasco PTC-423S/15 temperature control system (Jasco, Tokyo, Japan). Results obtained were baseline corrected, smoothened and presented as mean residue molar ellipticity [θ]MRW at a given wavelength. For normalizing CD signals protein concentration was determined by amino acid analysis.

### Fourier transformed infrared spectroscopy (FTIR)

For FTIR measurements 30 µL of rDer p-8x in 10 mM sodium phosphate buffer pH 7.4 with the infrared spectrometer Tensor27 (Bruker, Billerica, MA, USA) and the analyzing AquaSpec cell (MicroBiolytics, Esslingen, Germany) was used. Background measurements were performed with water and 10 mM sodium phosphate buffer pH 7.4. For determination of secondary structural elements, the amide I band (1600-1700 cm-1) was evaluated with the Opus confocheck software (Bruker, Billerica, MA, USA).

### Dynamic Light Scattering

Protein solutions were added to an 802 DLS Quarz Cell, 12 µL (Viscotek, Malvern Instruments, Malvern, UK) and measured with the Viscotek 802 DLS instrument (Viscotek, Malvern Instruments, Malvern, UK). For evaluation of the data OmniSize3.0 software (Viscotek, Malvern Instruments, Malvern, UK) was used.

### Proteolytic stability

Proteolytic stability was examined by degradation of the protein over time. In short, micro-/lysosomal fractions from the murine cell line JAWS II were obtained as previously described (Egger et al., PLoS One. 2011 Feb 16;6(2):e17278). 7 µg of mircrosomes were mixed with 5 µg of protein in the presence of digestion buffer (0.1 M citrate buffer pH 4.8, 2 mM dithiothreitol (Applichem, Darmstadt, Germany) and incubated for various time points at 37°C. The reaction was stopped by putting the reactions on 95°C for 5 min. Samples were stored at -20°C until examination of the degradation on SDS-PAGE.

### ANS-displacement assay

Binding ability of the protein to a hydrophobic molecule was determined by ANS (8-anilinonaphthalene-1-sulfonic acid (Sigma-Aldrich, St. Louis, MO; USA) displacement assay. The protein was mixed with different ratios of sodium-deoxycholate (Applichem, Darmstadt, Germany) in 10 mM sodium phosphate buffer pH 7.4 and incubated at 25°C for 1 hour. 75 µL of the sample were transferred to a 96 well plate (Greiner BioOne, Kremsmünster, Austria) and incubated with 25 µL of 200 µM ANS solution for 5 min at 25°C. Fluorescence of the samples was measured with an excitation wavelength of 370 nm and emission spectrum was determined at 400-600 nm.

### ELISA experiment

For IgE ELISA experiments, Nunc Maxisorp 96 well plates (Nunc, Thermo Fisher Scientific Inc., Waltham, MA, USA) were coated with 2 µg/mL of protein in 50 µL of PBS, pH 7.4 (Applichem, Darmstadt, Germany) and incubated overnight at 4°C. Plates were washed with TBS, pH 7.4, 0.05% Tween (Applichem, Darmstadt, Germany). Wells were blocked with TBS, pH 7.4, 0.05% Tween 20, 0.5% (w/v) BSA (Applichem, Darmstadt, Germany) and incubated with 50 µL of patients' sera diluted 1:10 overnight at 4°C. Plates were washed in TBS, pH 7.4, 0.05% Tween 20 and bound IgE was detected with alkaline phosphatase-conjugated mouse anti-human IgE antibodies (BP Pharmingen, San Diego, CA, USA), after incubation for 1 hour at 37°C and 1 hour at 25°C. As substrate 10 mM 4-Nitrophenyl phosphate (Applichem, Darmstadt, Germany) was used and OD was measured at 405/492 nm. Results are shown as median of OD values of all subjects tested.

### Mediator release assays using human sera

For mediator release assays 1x10⁵ rat basophil leukemia cells (RBL-2H3) in 50 µL medium (MEM with Earls salts (PAN Biotech, Aigenbach, Germany), 5% FCSi (PAN Biotech, Aigenbach, Germany), 4 mM L-glutamine (PAN Biotech, Aigenbach, Germany) were seeded in a 96 well Nunclone (Thermo Scientific, Waltham, MA, USA) plate. Patients' sera were pre-incubated with AG-8 cells (ATCC, Wesel, Germany) diluted 1:2 in medium for 1 hour at 37°C. 50 µL of serum without cells was added to each well and incubated overnight at 37°C, 7% CO₂. Cells were washed with Tyrode's buffer (Tyrode's salt (Sigma), NaHCO₃ (Applichem, Darmstadt, Germany), BSA (Applichem, Darmstadt, Germany) and incubated with 100 µL of serial antigen dilutions in Tyrode's buffer and deuterium water 1:2 for 1 hour at 37°C, 7% CO₂. For maximal release, cells were lysed with 10 µL 10% Triton X-100 (Amresco, Solon, OH, USA). For the detection of β-hexosaminidase, 50 µL of supernatant was transferred to a fresh 96 well plate (Greiner BioOne, Kremsmünster, Austria) and incubated with citrate buffer (Applichem, Darmstadt, Germany), 0.1 M, pH 4.5 and 160 µM 4-methyl umbelliferyl-N-acetyl-beta-D-glucosaminide (Sigma-Aldrich, St. Louis, MO, USA) for 1 hour at 37°C. The reaction was stopped with 100 µL of 0.2 M glycine buffer pH 10.7 (glycine (Applichem, Darmstadt, Germany), sodium chloride (Applichem, Darmstadt, Germany) and the fluorescence was measured at 465 nm with an excitation of 360 nm. Results are shown as median of ng of protein needed for half maximal release.

### Immunization study

To determine the immunological behavior *in vivo,* female BALB/c mice were immunized subcutaneously into the back with 5 µg of protein adsorbed to aluminum hydroxide. Cross-reactivity with the parental protein Der p 2 was determined by IgE crosslinkage, induction of cross-reactive IgG antibodies as well as cytokine profiling.

### Mediator release assay using murine sera

For mediator release assays 4x10⁴ rat basophil leukemia cells (RBL-2H3) in 100 µL medium (MEM with Earls salts (PAN Biotech, Aigenbach, Germany), 5% FCSi (PAN Biotech, Aigenbach, Germany), 4 mM L-glutamine (PAN Biotech, Aigenbach, Germany), 2 mM sodium pyruvate (PAA), 10 mM HEPES (PAA), 10 µM βmercaptoethanol (Applichem, Darmstadt, Germany), 1% Pen/Strep (PAN)) were seeded in a 96 well Nunclone (Thermo Scientific) plate and incubated overnight at 37°C, 5% CO₂. Mouse sera were added to a final dilution of 1:25 and incubated at 37°C, 5% CO₂ for 2 hours. Cells were washed with Tyrode's buffer (Tyrode's salt (Sigma), NaHCO₃ (Applichem, Darmstadt, Germany), BSA (Applichem, Darmstadt, Germany) and incubated with 100 µL of serial protein dilutions in Tyrode's buffer for 30 min at 37°C, 5% CO₂. For maximal release, cells were lysed with 10 µL 10% Triton X-100 (Amresco, Solon, OH, USA). For the detection of β-hexosaminidase, 50 µL of supernatant was transferred to a fresh 96 well plate (Greiner BioOne, Kremsmünster, Austria) and incubated with citrate buffer, 0.1 M, pH 4.5 and 160 µM 4-methyl umbelliferyl-N-acetyl-beta-D-glucosaminide (Sigma Aldrich, St. Louis, MO, USA) for 1 hour at 37°C. The reaction was stopped with 100 µL of 0.2 M glycine buffer pH 10.7 (glycine (Applichem, Darmstadt, Germany), sodium chloride (Applichem, Darmstadt, Germany) and the fluorescence was measured at 465 nm with an excitation of 360 nm. Results are shown as median of ng of protein needed for half maximal release.

### IgG ELISA with murine sera

For IgG ELISA experiments, Maxisorp 96 well plates (Nunc, Thermo Fisher, Waltham, MA, USA) were coated with 2 µg/mL of protein in 50 µL of PBS, pH 7.4 and incubated overnight at 4°C. Plates were washed with TBS, pH 7.4, 0.05% Tween. Wells were blocked with TBS, pH 7.4, 0.05% Tween 20, 0.5% (w/v) BSA and incubated with 100 µL of serial-diluted mouse sera overnight at 4°C. Plates were washed in TBS, pH 7.4, 0.05% Tween 20 and bound IgG1 and IgG2a was detected with alkaline phosphatase-conjugated rat anti-mouse IgG1/IgG2a antibodies (Southern Biotech, Birmingham, AL, USA), after incubation for 2.5 hours at 25°C. As substrate 10 mM 4-Nitrophenyl phosphate (Applichem, Darmstadt, Germany) was used and OD was measured at 405/492 nm. Results are shown as median of endpoint titers of all groups tested.

### ELISpot

ELISpot plates (Merck, Millipore, Darmstadt, Germany) were activated with 35% ethanol for 10 min at 25°C. After washing of the membrane with PBS, it was coated with 50 µL of 4 µg/mL capture antibody (eBioscience, Affymetrix, Santa Clara, CA, USA and incubated over night at 4°C. The membrane was blocked with Proliferation medium (MEM with Earls salts, 5 mL L-glutamine , 1% FCSi , 2.5% Pen/Strep (all PAN Biotech, Aigenbach, Germany), 5 mL sodium-pyruvate, 10 mL HEPES (both PAA, GE Healthcare, Pasching, Austria), 5 mL non-essential amino acids (Sigma Aldrich, St. Louis, MO, USA), 1 µL β-mercaptoethanol (Applichem, Darmstadt, Germany) containing 5 % FCSi for 3.5 hours in a humidified chamber. Wells were washed with PBS, 50 µL of protein solution (40 µg/mL) and 1x105 splenocytes from mice were added to each well. Plates were incubated at 37°C, 7% CO2 for 36 hours. After washing of the wells with PBS and PBS, 0.1% Tween 20, 0.1 µg of biotinylated detection antibody (eBioscience, Affymetrix, Santa Clara, CA, USA) in PBS, 1% BSA was added and incubated for 2.5 hours at 25°C in a humidified chamber. Plates were washed with PBS, 0.1% Tween 20. For detection streptavidin-HRP conjugate in PBS, 1% BSA was added to each well and incubated for 2 hours at 25°C in a humidified chamber. After extensive washing of the membrane, substrate solution (substrate buffer (0.09 M citric acid (Applichem, Darmstadt, Germany), 0.16 M Na2HPO4 (Applichem, Darmstadt, Germany) mixed to pH 5.0), 20 mM AEC (3-Amino-9-ethylcarbazole) solution (1:15) (Sigma Aldrich, St. Louis, MO, USA) and H2O2 (1:1500) (Merck, Darmstadt, Germany) was added until spots are visible, the reaction was stopped with water. Dried membrane was scanned and spots were counted. Results show number of cells per well background subtracted.

### Results

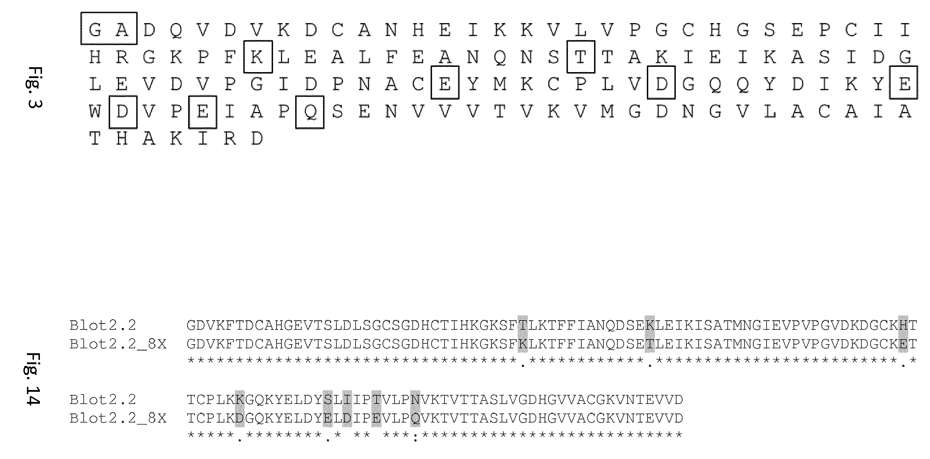

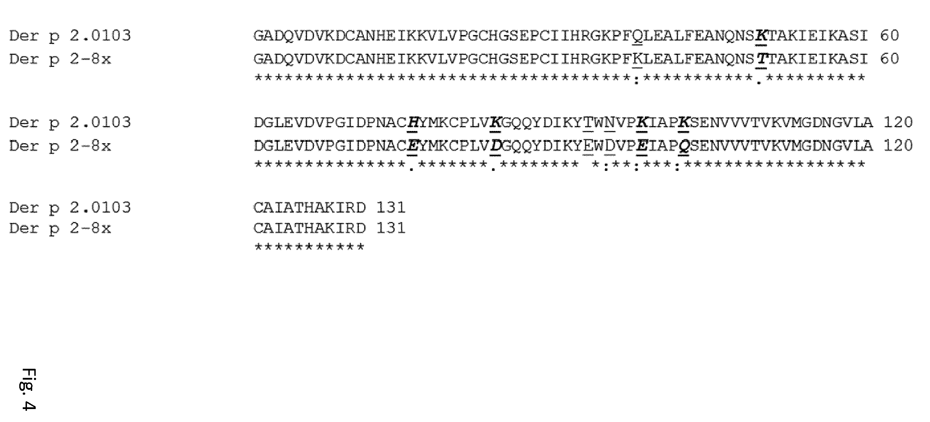

In the present example the design of hypoallergenic variants of the group 2 allergen from *Dermatophagoides pteronyssinus* by *in silico* mutagenesis is described. Polar or charged amino acid residues which were surface accessible were exchanged to either charged or polar amino acid residues. As the overall structure of Der p 2 should not be altered, the variant was, after molecular modelling, evaluated by its energetic stability regarding the Z-score by using the program ProSA-2003 (Sippl M.J, Proteins 17:355-362, 1993). Concerning molecular modeling, the Der p 2 Mod sequence differed besides the N-terminal Gly-Ala extension in five positions from the template sequence 1KTJ, in particular at residue D3S, Q4E, L42V, S49T and N116D, here the sequence numbering refers to Der p 2 Mod. The overall C-alpha root-mean-square deviation (rmsd) between 1KTJ and Der p 2 Mod is 0.14 Å. The respective ProSA-2003 (Sippl M.J, Proteins 17:355-362, 1993) Z-scores were -8.04 (1KTJ) and -8.17 (Der p 2 Mod). These data indicated that the structural model of Der p 2 Mod was energetically favorable. After inducing 8 point mutation on the surface of the protein (Fig. 3) 6 possible mutants with numerical equivalent Z-score values were obtained (see Table 1). As the side chain size change is larger for K->D than K->E, the second hit (Q38K, K50T, H76E, K84D, T93E, T95D, K98E, K102Q; "Der p 2-8x") was selected for the further experiments. Including the Gly-Ala expression tag, the resulting molecule (Z-score: -8.98) scored better than the wildtype protein (-8.04) by 0.94 arbitrary units, indicating a stable mutant protein. A sequence alignment between Der p 2.0103 and Der p 2-8x is shown in fig. 4.

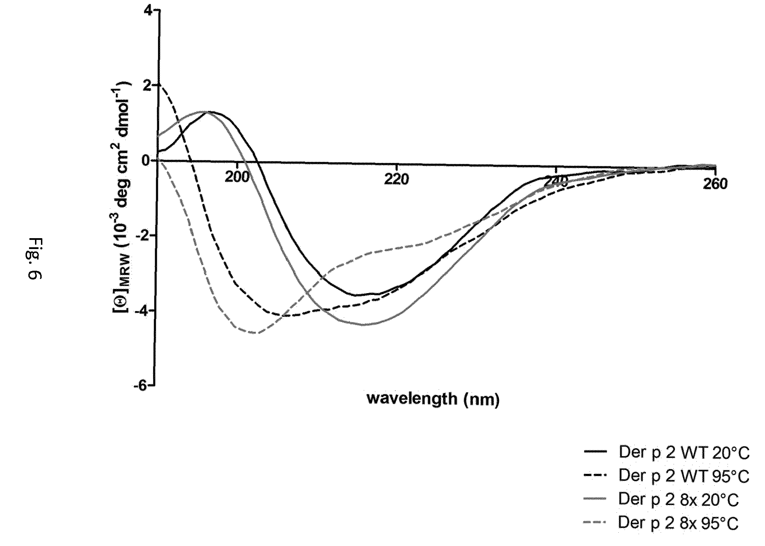

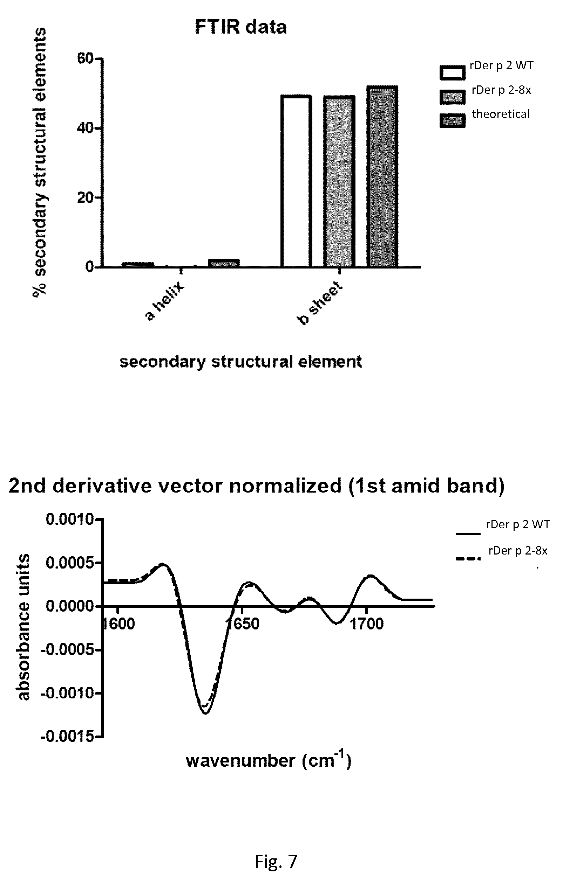

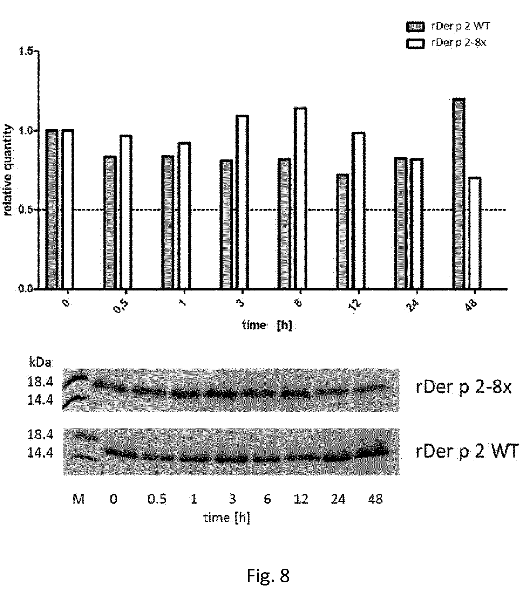

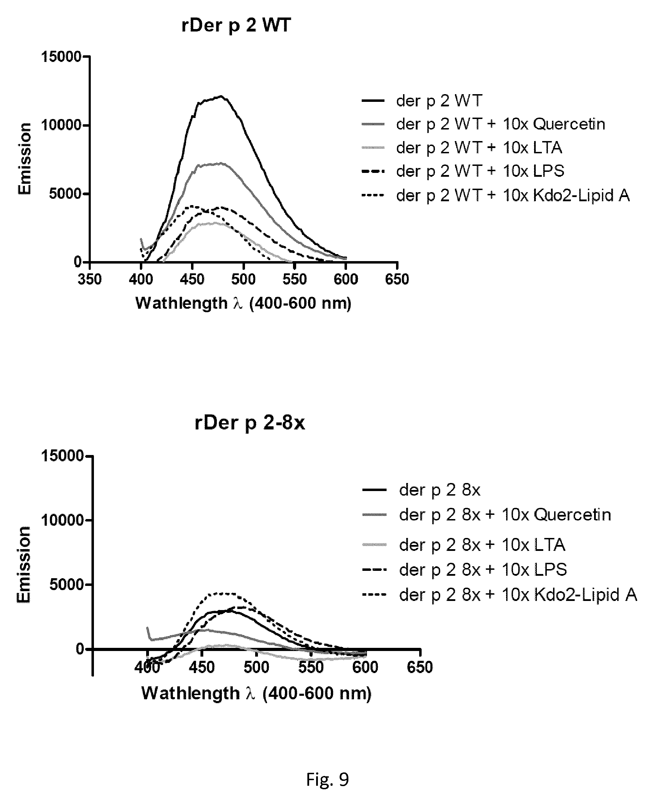

The protein was expressed in the *E. coli* Shuffle strain and purified from the soluble fraction by ion exchange chromatography (see Fig. 5). To investigate the overall structure of rDer p 2-8x circular dichroism (CD) measurements were performed, which did not depict any changes secondary structure elements of the mutant protein in comparison to the wildtype molecule rDer p 2 (Fig. 6). These results were confirmed by FTIR measurements, as the variant molecule not only showed the same amount of secondary structural elements as the wildtype protein rDer p 2, but also the amide I band was identical (Fig. 7). The identity, as well as the correct formation of the disulfide bonds of the protein was determined by mass spectrometry. Also, the variant rDer p 2-8x depicted a similar hydrodynamic radius as natural and recombinant rDer p 2 wildtype, as revealed by dynamic light scattering experiments. Further all proteins did not form aggregates, which can also be taken as an indicator for correct folding. Overall, rDer p 2-8x revealed similar structural properties as the wild type protein. Proteolytic stability was tested by incubation with the lysosomal fraction of JAWS II cells. Even after 48 hours of incubation, more than 50% of the original amount of protein was intact (Fig. 8). In ANS displacement assays, several putative ligands were tested (Fig. 9). Here, it appears that rDer p 2-8x behaves differently to the wildtype rDer p 2.

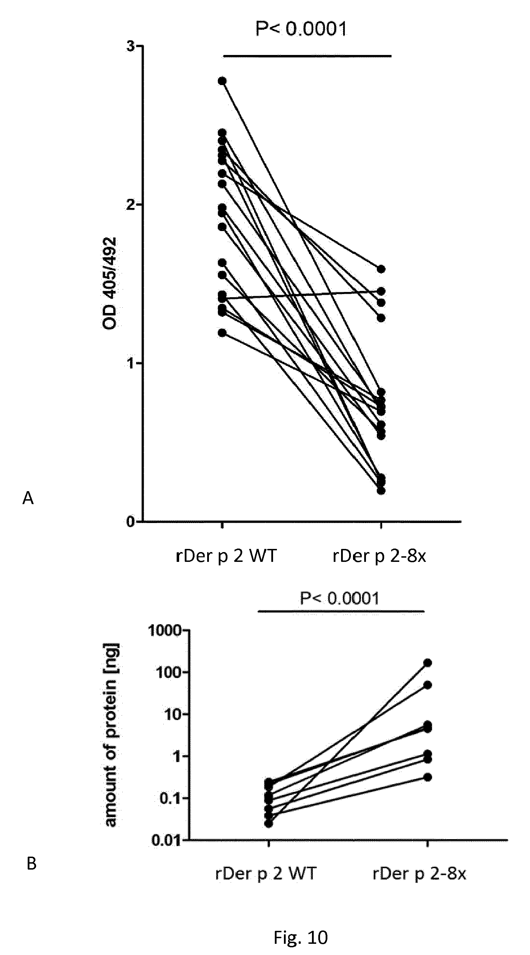

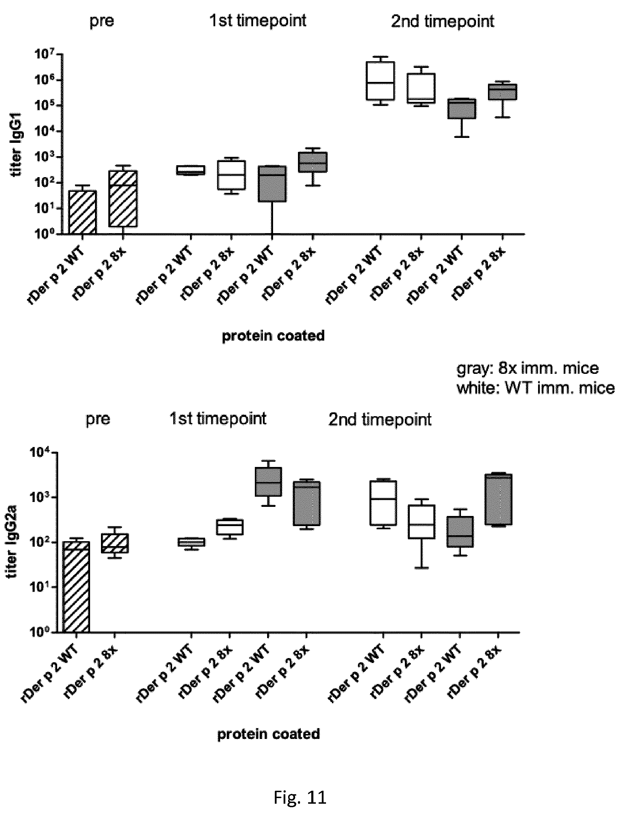

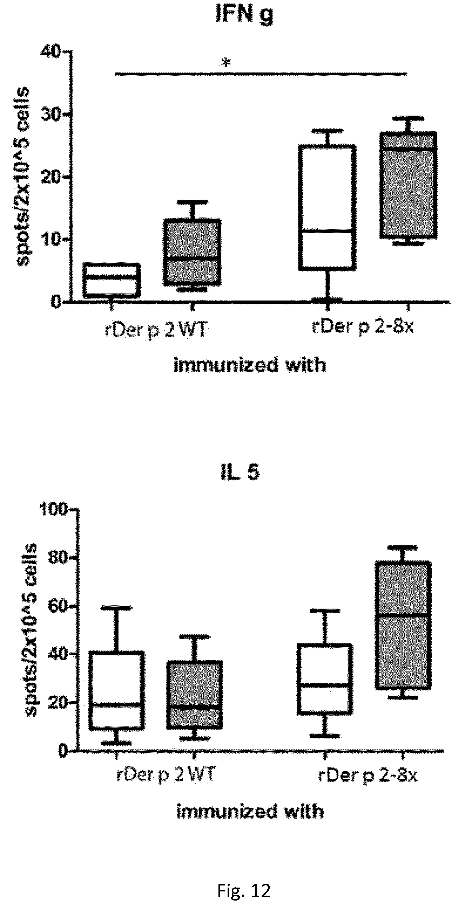

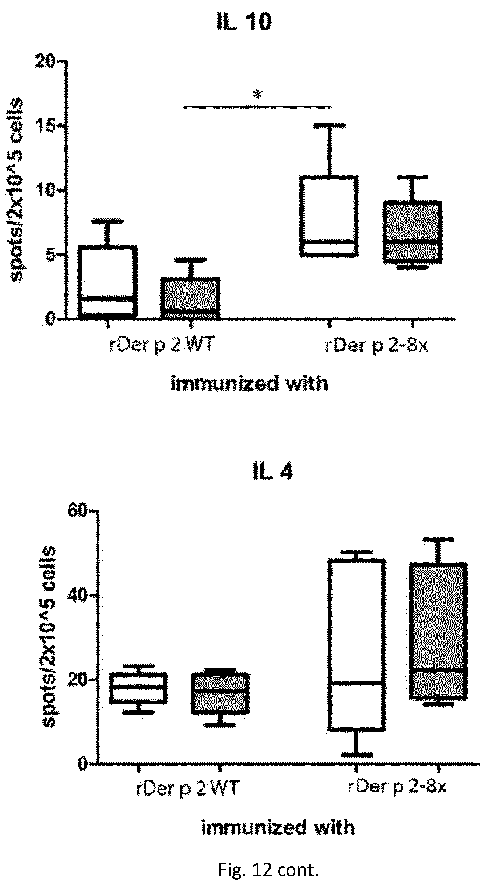

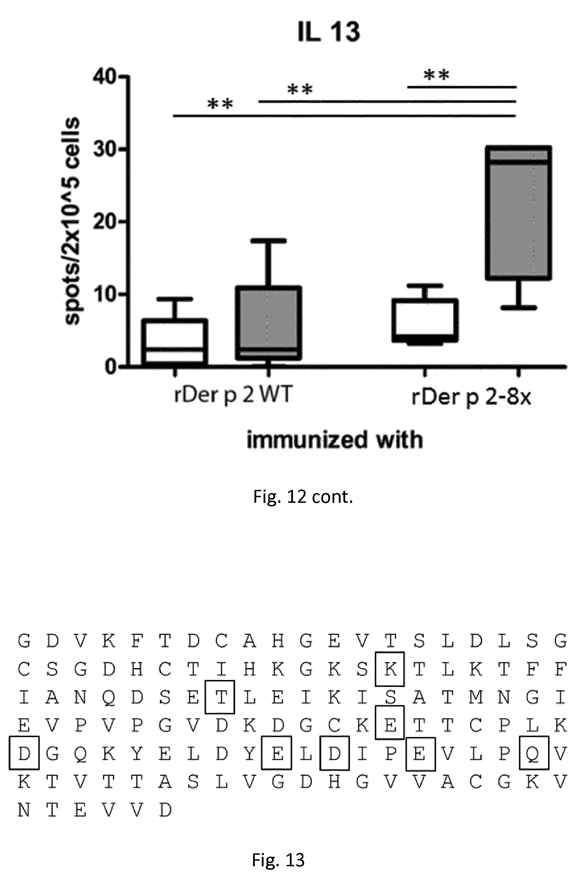

As reduced IgE-binding is essential for save allergen immunotherapy, rDer p 2-8x was tested for its IgE cross-reactivity by IgE ELISA experiments and mediator release assays. For the ELSIA with patients' sera rDer p 2-8x showed significantly reduced IgE-binding in comparison to its recombinantly produced parental allergen (Fig. 10A). The same was true for mediator release assays, where rDer p 2-8x in contrast to recombinantly produced rDer p 2 depicted a mean reduction in IgE-binding capacity of 244-fold, reaching from 11 fold to non-responder when serum of patients was tested (Fig. 10B). Despite the 8 point mutations on the molecule, various experiments suggested a very similar folding of rDer p 2-8x in comparison to rDer p 2 wildtype. Whereas due to these mutations it was shown in IgE cross-reactivity studies, that the variant protein reveals a significantly reduced IgE-binding capacity in comparison to the wildtype protein. Further, the immunological behavior of Der p 2-8x was tested in a mouse model. Therefore, female BALB/c mice were immunized subcutaneously with 5 µg protein adsorbed to aluminum hydroxide; mice were immunized at day 0, 7 14 and 21 and blood samples of said mice were collected at day 0, 14 and 28. Despite the mutations on the surface, it could be shown in ELISA experiments that rDer p 2-8x is able to induce IgG1 as well as IgG2a antibodies cross-reactive with the WT allergen (Fig. 11). Also the IgE antibodies induced by our immunization schema were cross-reactive between rDer p 2 WT and rDer p 2-8x. Cytokine levels secreted from splenocytes were evaluated in ELISpot assays (Fig. 12). In general, levels of TH2 cytokines, IL-4, IL-5, IL-13 did not change significantly, except when mice were immunized with rDer p 2-8x, and splenocytes were restimulated with rDer p 2-8x, which induced significantly higher levels of IL-13. IFN-γ levels, were slightly increased when mice were immunized with rDer p 2-8x. The same holds true for IL-10 levels. In summary, the cytokine data suggests cross-reactivity on T cell level.

### Example 3: Blo t 2 variants comprising up to eight point mutations at T35, K48, H74, K81, S90, I92, T95, and N99 of wild-type Blo t 2

In the tropical climate zone, storage mites, as *Blomia tropicalis* are a major risk factor to develop respiratory allergies, i.e. rhinitis or asthma. Most studies have been performed in middle and southern America or south-east Asia, as sensitization rates to *Blomia tropicalis* are high in those areas. Blo t 2 resembles a structural homolog to the house dust mite allergen Der p 2 of *Dermatophagoides pteronyssinus.* While sequence identity of both allergens is below 40%, the cross-reactivity between them has not been studied extensively and is still under discussion. The allergen Blo t 2 from *Blomia tropicalis* is listed as a minor allergen, but resent results from our group suggest a much higher sensitization prevalence to Blo t 2 as reported in the literature. As allergen-specific immunotherapy is the only curative approach to treat allergies, a low-IgE binding variant of Blo t 2 following the method developed for the design of Der p 2-8x was designed.

### Computer assisted rational design of point mutations in Blo t 2 and its homologs

For the design of Blo t 2.2-8x, the design template of Der p 2-8x was used. Mutation sites were evaluated by ProSA-2003 (Sippl M.J, Proteins 17:355-362, 1993). As template the isoform Blo t 2.2 (UniProt Acc. No. A6XEN9), termed Blo t 2.2 in the following, was used.

### Patients' sera

Storage mite allergic patients from Brazil, all sensitized to Blo t 2 from *Blomia tropicalis,* were selected based on typical case history and preliminary IgE ELISA experiments.

### Expression of Blo t 2.2-8x

Expression plasmids carrying a nucleic acid sequence encoding Blo t 2.2-8x were transformed in *E. coli* Shuffle™ T7 Express (New England Biolabs, Ipswich, MA, USA) and grown at 37°C to an OD₆₀₀ of 0.6 in LB supplemented with 10 mL/L glycerol, 2 mM MgSO₄, 2 g/L ammonium sulfate, 10 mM NaP-buffer, pH 7.4, 25 mg/L kanamycin. The protein production was induced by adding 0.5 mM iso-prpoy-β-D-thiogalactopyranoside (IPTG) (Applichem, Darmstadt, Germany) and the culture was incubated for 4 hours at 37°C under constant shaking. After the cells were harvested by low speed centrifugation, the bacterial cell pellet was resuspended in appropriate lysis buffer and the supernatant of the bacterial lysate was used for further experiments.

### SDS-PAGE and immunoblots

*E. coli* lysates and purified proteins (i.e. Blo t 2.2) were analyzed by denaturing sodium dodecyl polyacrylamide gel electrophoresis (SDS-PAGE) using 15% gels. Proteins were visualized by staining with Coomassie Brilliant Blue R-250 (Biorad, Hercules, CA, USA).

### Protein quantification in bacterial extract

Total protein content of the lysate sample was determined by Bradford assay using a standard curve. Further, Coomassie-stained SDS-gels were scanned with a Light imager (BioRad, Hercules, CA, USA) and protein bands were quantified relatively to a reference band (purified Blo t 2.2) using the Image Lab software (BioRad, Hercules, CA, USA).

### Immunoblots

For immunoblot analysis 0.5 µg of the protein of interest were loaded on a protein gel and subsequently proteins were separated by SDS-PAGE and blotted semidry on nitrocellulose membrane (GE healthcare, Whatmann, Little Chalfont, UK). A pool of patients' sera (n= 10) was added in a 1:10 dilution in blocking buffer and incubated at 4°C over night. For the detection of bound IgE, an alkaline phosphatase conjugated mouse anti-human IgE antibody (BD Pharmingen, San Diego, CA, USA) was added. Nitro blue tetrazolium chloride (NBT)/ 5-bromo-4 chloro-3-indolyl phosphate (BCIP) (both Applichem, Darmstadt, Germany) in buffer was added for colorimetric detection.

### ELISA experiment

For IgE ELISA experiments, Nunc Maxisorp 96 well plates (Nunc, Thermo Fisher Scientific Inc., Waltham, MA, USA) were coated with 2 µg/mL of protein of interest in 50 µL of PBS, pH 7.4 (Applichem, Darmstadt, Germany) and incubated overnight at 4°C. Plates were washed with TBS, pH 7.4, 0.05% Tween (Applichem, Darmstadt, Germany). Wells were blocked with TBS, pH 7.4, 0.05% Tween 20, 0.5% (w/v) BSA (Applichem, Darmstadt, Germany) and incubated with 50 µL of single patients' sera diluted 1:10 overnight at 4°C. Plates were washed in TBS, pH 7.4, 0.05% Tween 20 and bound IgE was detected with alkaline phosphatase-conjugated mouse anti-human IgE antibodies (BP Pharmingen, San Diego, CA, USA), after incubation for 1 hour at 37°C and 1 hour at 25°C. As substrate 10 mM 4-nitrophenyl phosphate (Applichem, Darmstadt, Germany) was used and OD was measured at 405/492 nm. Results are shown as mean of OD values of all subjects tested.

### Results

The aim of the present example was to design a hypoallergenic variant of the group 2 allergen from *Blomia tropicalis* by *in silico* mutagenesis and test its properties in wet lab. Using Der p 2-8x as design template (see examples 1 and 2), the corresponding amino acids of Blo t 2.2 were mutated to generate the variant Blo t 2.2-8x. Using ProSA-2003 (Sippl M.J, Proteins 17:355-362, 1993) for *in silico* evaluation of protein stability, calculated Z-score suggested an overall stable protein. In analogy to Der p 2-8x, the amino acids T35K, K48T, H74E, K81D, S90E, I92D, T95E, and N99Q of Blo t 2.2 were mutated (see Fig. 13), resulting in a protein with calculated Z-score of -8.09, whereas the parental allergen Blo t 2.2 (UniProt Acc. No. A6XGEN9) had a score of -7.26, resulting in a delta Score of -0.83. These data indicated that the structural variant of Blo t 2.2, Blo t 2.2-8x, was highly stable. Fig. 14 shows a sequence alignment of Blo t 2.2 with the mutant Blo t 2.2-8x.

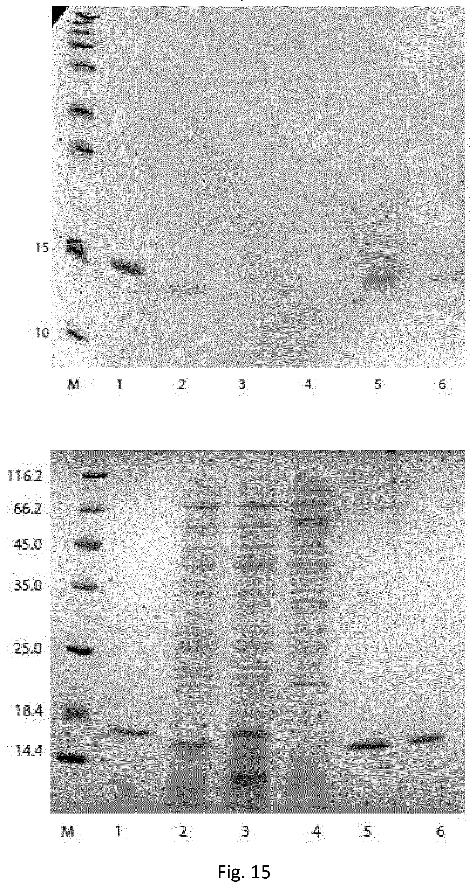

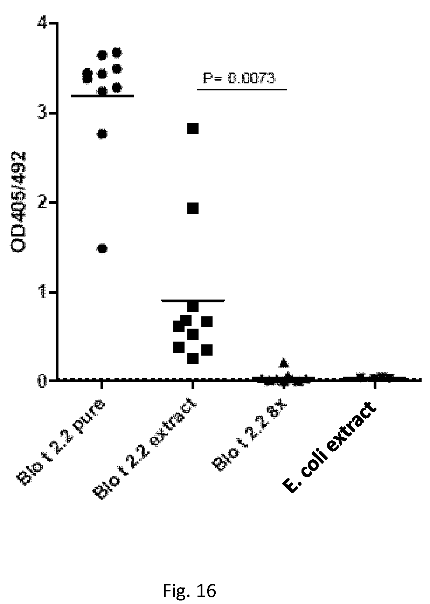

The protein was expressed in the *E. coli* Shuffle (New England Biolabs, Ipswich, MA, USA). Bacterial lysates were used to determine the IgE-binding capacity of Blo t 2.2-8x and compare it to the parental allergen Blo t 2.2. Therefore, immunoblot and ELISA experiments were performed. For immunoblot assays, 0.5 µg protein were loaded on a gel and the IgE binding of Blo t 2.2-8x was diminished for the pool of Blo t 2 allergic patients' sera (n= 10) (Fig. 15). This observation was verified by IgE ELISA experiments (Fig. 16), where a significant reduction of IgE-binding to Blo t 2.2-8x compared to Blo t 2.2 was found.

## Claims

1. A variant of a polypeptide of the Niemann-Pick type C2 (NPC2) protein family comprising a motif consisting of the amino acid sequence NQX₁X₂X₃X₄X₅PGX₆X₇X₈CX₉X₁₀CPX₁₁X₁₂GX₁₃YX₁₄X₁₅X₁₆PX₁₇X₁₈X₁₉PX₂₀ (SEQ ID No. 1), wherein X₁ is N or D, X₂ is S or T, X₃ is K, N, A, P or E, X₄ is a peptide consisting of 16 or 17 amino acid residues, X₅ is V or I, X₆ is a peptide consisting of 3 amino acid residues, X₇ is N or D, X₈ is A, L or G, X₉ is H or K, X₁₀ is a peptide consisting of 3 amino acid residues, X₁₁ is a peptide consisting of 2 amino acid residues, X₁₂ is K, X₁₃ is a peptide consisting of 6 amino acid residues, X₁₄ is T, S, A, D, N or NI, X₁₅ is a peptide consisting of 2 amino acid residues or G, L, Y or W, X₁₆ is N, V, T, L, I, S or E, X₁₇ is K, R, A, Q or T, X₁₈ is I, L, V, N or A, X₁₉ is I, L, T or A and X₂₀ is K, N, S, T or E, wherein at least one amino acid residue selected from the group consisting of X₃, X₉, X₁₂, X₁₇ and X₂₀ is mutated.

2. Variant according to claim 1, wherein the mutation is a substitution or deletion.

3. Variant according to claim 1 or 2, wherein X₃ of the variant is T, P, C, Y, V, S, W, F, I, N, or G, preferably T, W, Y, P, C, V, S, I, N or G, more preferably T.

4. Variant according to any one of claims 1 to 3, wherein X₉ of the variant is E, Q or D, preferably E.

5. Variant according to any one of claims 1 to 4, wherein X₁₂ of the variant is D, N, E, P, S, Q, G or T, preferably D or E, more preferably D.

6. Variant according to any one of claims 1 to 5, wherein X₁₇ of the variant is E, D, N, Q, or G, preferably E or D, more preferably E.

7. Variant according to any one of claims 1 to 6, wherein X₂₀ of the variant is , Q, W, M, E, L, Y, N, T, S, I, F, D, or G, preferably Q.

8. Variant according to any one of claims 1 to 7, wherein X₁₄ and/or X₁₆ of SEQ ID No. 1 are mutated, preferably by substitution or deletion.

9. Variant according to any one of claims 1 to 8, wherein X₁₄ of the variant is E, K or D, preferably E.

10. Variant according to any one of claims 1 to 9, wherein X₁₆ of the variant is D, E, K, or R, preferably E or D.

11. Variant according to any one of claims 1 to 10, wherein the motif consists of an amino acid sequence which is at least 90% identical to an amino acid sequence selected from the group consisting of SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11 and SEQ ID No. 12.

12. Variant according to any one of claims 1 to 11, wherein the polypeptide of the Niemann-Pick type C2 (NPC2) protein family comprises a second motif consisting of the amino acid sequence X₂₁HX₂₂GX₂₃X₂₄X₂₅X₂₆X₂₇ (SEQ ID No. 13), wherein X₂₁ is L or I, X₂₂ is R or K, X₂₃ is K, T or E, X₂₄ is K, P, A, E or S, X₂₅ is F, L or M, X₂₆ is Q, K, A, T, S or D and X₂₇ is L or M.

13. Variant according to claim 12, wherein X₂₆ of the variant is H, K, R, D, or E, preferably K.

14. Variant according to claim 12 or 13, wherein the second motif consists of an amino acid sequence which is at least 80% identical to an amino acid sequence selected from the group consisting of SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23 and SEQ ID No. 24.

15. Variant according to any one of claims 1 to 14, wherein the polypeptide of the Niemann-Pick type C2 (NPC2) protein family comprises an amino acid sequence which is at least 90% identical to an amino acid sequence selected from the group consisting of SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34 and SEQ ID No. 35.

16. Variant according to any one of claims 1 to 15 for the use in the treatment or prevention of allergies caused by polypeptide of the Niemann-Pick type C2 (NPC2) protein family.

17. Pharmaceutical preparation comprising a variant according to any one of claims 1 to 16.
